# EUROPÄISCHE PATENTSCHRIFT

(11) **EP 3 419 708 B1**
(45) Veröffentlichungstag und Bekanntmachung des Hinweises auf die Patenterteilung: **20.11.2019**
(21) Anmeldenummer: 17708680.8
(22) Anmeldetag: 22.02.2017
(51) Int. Cl.: A61M 15/00

(54) **INHALATOR**
INHALER
INHALATEUR

(30) Priorität: 24.02.2016 EP 16020052; 20.02.2017 WO PCT/EP2017/025029
(43) Veröffentlichungstag der Anmeldung: 02.01.2019
(73) Patentinhaber: Boehringer Ingelheim International GmbH, 55216 Ingelheim am Rhein (DE)
(72) Erfinder: HOLAKOVSKY, Holger, 55216 Ingelheim am Rhein (DE); BESSELER, Jens, 55216 Ingelheim am Rhein (DE); FRENTZEL-BEYME, Jessica, 55216 Ingelheim am Rhein (DE); HERRMANN, Frank, 55216 Ingelheim am Rhein (DE); KAEMPER, Markus, 55216 Ingelheim am Rhein (DE)
(74) Vertreter: Simon, Elke Anna Maria
(86) Internationale Anmeldenummer: PCT/EP2017/025032
(87) Internationale Veröffentlichungsnummer: WO 2017/144182

(56) Entgegenhaltungen:
- EP-A1- 0 528 764
- WO-A1-2005/089842
- WO-A1-2007/118648
- WO-A1-2011/073306
- WO-A1-2013/150021
- CN-U- 204 521 855
- CN-U- 204 890 849
- CN-U- 204 890 850
- JP-A- H09 262 295
- US-A1- 2010 294 278

## Beschreibung

Die vorliegende Erfindung betrifft einen Inhalator gemäß dem Oberbegriff des Anspruchs 1.

Die vorliegende Erfindung betrifft insbesondere einen Inhalator zur Ausgabe bzw. Inhalation einer vorzugsweise pulverförmigen Formulierung, also einen Pulverinhalator.

Bei der Formulierung handelt es sich insbesondere um ein therapeutisches Mittel bzw. Arzneimittel. Insbesondere enthält die Formulierung dementsprechend mindestens einen Wirkstoff oder besteht daraus. Die Formulierung dient also insbesondere der medizinischen Behandlung oder sonstigen therapeutischen Zwecken.

Bei der vorliegenden Erfindung ist die Formulierung in Kapseln aufgenommen, wobei jede Kapsel eine Dosis der Formulierung enthält. Die Formulierung ist also in die Kapseln vordosiert.

Bei der vorliegenden Erfindung sind unter dem Begriff "Kapsel" primär Behältnisse mit einer festen oder einer zumindest im wesentlichen starren, insbesondere dichten, einstückigen, geschlossenen und/oder durchgängigen Hülle zu verstehen, die insbesondere separat voneinander handhabbar und/oder öffenbar sind.

Vorzugsweise sind in einem weiteren Sinn gemäß der vorliegenden Erfindung unter dem Begriff "Kapsel" auch sonstige Behältnisse, Verpackungen oder dergleichen mit jeweils einer Dosis der Formulierung zu verstehen, die insbesondere separat voneinander handhabbar und/oder öffenbar sind.

Die EP 0 147 755 A2 offenbart einen Inhalator für die Inhalation pulverförmiger Arzneimittel aus länglichen Kapseln. Der Inhalator weist eine Kapselkammer auf, in die jeweils eine Kapsel manuell einführbar ist. Die Kapsel wird durch manuelle Betätigung einer Öffnungseinrichtung in der Kapselkammer längsseitig angestochen und dadurch geöffnet. Beim Inhalieren führt ein durch die Kapselkammer strömender Luftstrom dazu, dass die Kapsel in der Kapselkammer hin- und herbewegt wird, wobei das pulverförmige Arzneimittel ausgetragen und im Luftstrom dispergiert wird. Die vorliegende Erfindung benutzt insbesondere dieses Prinzip, ist jedoch aber auch bei sonstigen Techniken zum Austrag einer Formulierung einsetzbar.

Die WO 2005/049121 A1 offenbart einen Pulverinhalator mit einer Vielzahl von Kapseln. Die zylindrischen Kapseln sind aufrecht hintereinander von einer Schiene geführt oder kettenartig miteinander verbunden.

Die WO 2005/089842 A1 offenbart einen Inhalator, mit dem eine pharmazeutische Pulverzubereitung aus einer aus zwei miteinander verbundenen Kapselteilen zusammengesetzten Kapsel dadurch zur Inhalation aus einem Saugraum zur Verfügung gestellt wird, dass der Inhalator eine Einrichtung zum Auseinandersprengen der lösbar miteinander verbundenen Kapselteile aufweist. Der Inhalator weist eine Zuführeinrichtung auf, mit der die einzelnen Kapseln aus einem Kapselvorrat in den Saugraum zugeführt werden. Die Zuführeinrichtung umfasst dabei einen schwenkbaren Deckel, der über eine Kniehebelmechanik mit einer Kapselfördervorrichtung bewegungsgekoppelt ist, so dass beim Aufschwenken des Deckels eine Kapsel in den Saugraum gefördert wird. Die Kapselfördereinrichtung selbst umfasst eine Kapselaufnahme in Form eines Drehrads mit mehreren jeweils eine Kapsel aufnehmenden Abteilen sowie einen Auswerfer zum Transfer der Kapsel zum Saugraum.

Die WO 2007/118648 A1 offenbart einen Pulverinhalator, der insbesondere mehrere Kapselkammern mit darin aufgenommenen Kapseln aufweist, wobei jede Kapselkammer insbesondere nur einmalig verwendet wird. Die Kapseln und Kapselkammern können radial ausgerichtet sein, wobei die Auslassöffnungen der Kapselkammern von einer gemeinsamen Abdeckung abgedeckt sein können. Dabei können die Kapseln und Kapselkammern auch in zwei axial versetzten Ebenen angeordnet sein. Gemäß einer anderen Ausführungsform kann der Inhalator auch nur eine Kapselkammer zur einzelweisen Aufnahme von Kapseln nacheinander zu deren Entleerung bei der Inhalation aufweisen. Weiter kann eine Mundstückabdeckung beim Schwenken die Kapseln vorwärts und einzelweise in die Kapselkammer bewegen.

Die WO 2011/039307 A2 zeigt einen Inhalator für die Inhalation pulverförmiger Arzneimittel aus Kapseln mit einem wechselbaren Austauschrohr, das eine Kapselkammer mit einem sich anschließenden Ausgabekanal bildet, wobei in die Kapselkammer eine Kapsel voreingesetzt ist. Das Austauschrohr weist Öffnungen für Nadeln zum Ausstechen der jeweiligen Kapsel auf, wobei die Öffnungen von einer Membran selbsttätig verschlossen werden können.

Die US 2010/0294278 A1 zeigt einen Pulverinhalator mit einem drehbaren Blisterträger. In dem Blisterträger sind Blister, die jeweils eine Dosis eines pulverförmigen Medikaments enthalten, radial angeordnet. Zum Betätigen eines Hebels kann der Blisterträger gedreht werden. Die Blister sind einzeln radial in eine Ausgabeposition bewegbar, in der der Blister geöffnet wird und anschließend das Medikament vom Patienten inhaliert werden kann.

Eine Kapselkammer im Sinne der vorliegenden Erfindung ist vorzugsweise ein zumindest im Wesentlichen starres oder festes und/oder längliches Behältnis bzw. Kapselgehäuse mit einer insbesondere länglichen oder zylindrischen Kammer, in der die jeweilige Kapsel zur Entleerung insbesondere hin- und her bewegbar oder auf sonstige Weise bewegbar bzw. in Vibration oder Schwingung versetzbar ist. Insbesondere weist die Kapselkammer einen Einlass und Auslass an vorzugsweise entgegengesetzten Enden auf, so dass Luft durch die Kammer strömen kann, um die Kapsel in Bewegung bzw. Schwingung zu versetzen und/oder die Formulierung aus der Kapsel auszutragen.

Der vorliegenden Erfindung liegt die Aufgabe zugrunde, einen Inhalator anzugeben, der eine einfache Handhabung und/oder einen einfachen bzw. kompakten Aufbau gestattet.

Die obige Aufgabe wird durch einen Inhalator gemäß Anspruch 1 gelöst. Vorteilhafte Weiterbildungen sind Gegenstand der Unteransprüche.

Gemäß einem Aspekt der vorliegenden Erfindung ist eine dem Mundstück des Inhalators zugeordnete Abdeckung sowohl radial bewegbar als auch schwenkbar, so dass durch Schwenken der Abdeckung das Magazin von einer Kapsel zur nächsten Kapsel gefördert bzw. gedreht wird und dass durch radiales Bewegen der Abdeckung eine Kapsel radial in eine Ausgabeposition bewegt bzw. gefördert, insbesondere geschoben, wird. Dies ermöglicht eine sehr einfache Handhabung bei einem kompakten Aufbau, auch wenn viele Kapseln im Inhalator aufgenommen sind.

Insbesondere können also durch unterschiedliche Bewegungen der Abdeckung unterschiedliche Mechanismen ausgelöst werden. Vorzugsweise kann durch Schwenken der Abdeckung in eine Richtung und/oder in beide Richtungen das Magazin transportiert bzw. weiterbewegt oder gedreht werden. Vorzugsweise kann durch radiales Bewegen oder Ziehen der Abdeckung nach außen eine Kapsel oder Kapselkammer in eine Ausgabeposition bewegt werden. Insbesondere kann durch eine entgegengesetzte Bewegung oder Schließbewegung der Abdeckung die benutzte Kapsel oder Kapselkammer - vorzugsweise selbsttätig bzw. durch Federkraft - wieder zurück in eine Speicherposition bzw. Ausgangsposition bewegt oder geschoben werden.

Unter dem Begriff "radial" bzw. "radiale Bewegung" bzw. "radiale Richtung" im Sinne der vorliegenden Erfindung ist vorzugsweise eine Richtung bzw. Bewegung in eine Richtung von innen nach außen oder umgekehrt zu verstehen, insbesondere entlang eines Radius oder Durchmessers eines zumindest im Wesentlichen kreis-, platten- und/oder scheibenförmigen Bauteils, besonders bevorzugt eines Gehäuses oder Magazins des Inhalators. Vorzugsweise ist die radiale Bewegung bzw. ein radiales Schieben der Abdeckung als relative Bewegung der Abdeckung zum Gehäuse, Magazin und/oder Mundstück des Inhalators entlang eines Radius oder Durchmessers bzw. von innen nach außen oder umgekehrt zu verstehen.

Vorzugsweise ist das Mundstück des Inhalators in einem radialen Randbereich des Inhalators bzw. außen angeordnet. Insbesondere ist das Magazin mit den Kapseln radial weiter innen angeordnet als das Mundstück, vorzugsweise derart, dass die Kapseln durch eine radiale Bewegung vom Magazin in Richtung des Mundstücks bzw. von innen nach außen und/oder von außen nach innen bzw. zum Magazin bewegt werden.

Insbesondere wird durch eine radiale Bewegung der Abdeckung von innen nach außen bzw. von einer Mitte des Inhalators zum Mundstück eine Kapsel aus dem Magazin bzw. radial bzw. von innen nach außen bzw. in Richtung des Mundstücks bewegt.

Insbesondere wird durch eine radiale Bewegung der Abdeckung von außen nach innen bzw. vom Mundstück zu einer Mitte des Inhalators die verwendete Kapsel radial bzw. von außen nach innen bzw. in Richtung einer Mitte des Inhalators bzw. (zurück) zum Magazin bewegt.

Vorzugsweise ist unter dem Begriff "Schwenken" bzw. "Schwenkbewegung" eine Bewegung auf einer Kreisbahn zu verstehen. Insbesondere ist die Abdeckung bzw. sind die Schenkel dieser schwenkbar mit dem Gehäuse verbunden bzw. an oder in diesem gelagert, vorzugsweise in einer Mitte des Inhalators, insbesondere derart, dass die Abdeckung bzw. das äußere Ende dieser schwenkbar ist. Insbesondere ist durch Schwenken der Abdeckung das Mundstück wahlweise freigebbar oder abdeckbar.

Vorzugsweise ist die Abdeckung sowohl radial bewegbar als auch schwenkbar, wobei die Abdeckung durch eine radiale Bewegung nach außen anschließend schwenkbar ist und/oder durch eine radiale Bewegung nach innen anschließend drehfest bzw. in ihrer Drehlage festgelegt bzw. gesichert ist. Insbesondere ist die Abdeckung nicht gleichzeitig radial bewegbar und schwenkbar bzw. ist für die Schwenkbewegung eine vorangehende Radialbewegung nach außen erforderlich.

Gemäß einem weiteren Aspekt der vorliegenden Erfindung sind die Kapselkammern in dem Magazin vorzugsweise radial beweglich aufgenommen und einzelweise radial in eine Ausgabeposition bewegbar. Dies ist wiederum einem einfachen und kompakten Aufbau zuträglich, insbesondere wenn der Inhalator bzw. dessen Magazin eine Vielzahl von Kapseln und Kapselkammern aufnimmt, die jeweils nur einmalig benutzt werden.

Besonders bevorzugt ist nur eine Kapsel pro Kapselkammer vorgesehen. Dies ist insbesondere bei der Verwendung von klebrigen oder leicht agglomerierenden Pulvern vorteilhaft.

Grundsätzlich kann jedoch eine Kapselkammer auch für mehrere Kapseln bzw. mehrfach verwendet werden. Dies ermöglicht die Erhöhung der Anzahl der Kapseln, die in einem Inhalator oder Magazin unterbringbar sind.

Gemäß einer Ausführungsform werden die Kapseln vorzugsweise ohne zugeordnete Kapselkammer aus dem Magazin in die gemeinsame Kapselkammer bzw. Ausgabeposition bewegt. Dies gestattet einen besonders kompakten Aufbau des Magazins bzw. Inhalators und/oder die Unterbringung von besonders vielen Kapseln bei gleicher Baugröße.

Alternativ erfolgt das radiale Herausbewegen einer Kapsel vorzugsweise zusammen mit ihrer zugeordneten Kapselkammer aus dem Magazin. Dies gestattet mehr Raum für das Öffnen bzw. Anstechen der jeweiligen Kapsel in der Ausgabeposition bzw. Kapselkammer, als wenn die Kapseln in den Kapselkammern direkt im Magazin geöffnet bzw. angestochen werden würden. So wird eine möglichst kompakte Anordnung einer Vielzahl von Kapseln und Kapselkammern im Magazin ermöglicht. Dementsprechend wird insgesamt ein kompakter Aufbau des Inhalators und des Magazins ermöglicht.

Einzelne Aspekte, Merkmale, Eigenschaften und Vorteile der vorliegenden Erfindung ergeben sich aus den Ansprüchen und der folgenden Beschreibung bevorzugter Ausführungsformen anhand der Zeichnung. Es zeigt:
- Fig. 1: eine schematische Schnittdarstellung zum Funktionsprinzip eines vorschlagsgemäßen Inhalators;
- Fig. 2: eine perspektivische Ansicht eines vorschlagsgemäßen Inhalators gemäß einer ersten Ausführungsform mit geschlossener Abdeckung;
- Fig. 3: eine perspektivische Ansicht des Inhalators gemäß Fig. 2 mit radial herausgezogener Abdeckung;
- Fig. 4: eine perspektivische Ansicht des Inhalators gemäß Fig. 2 mit geöffneter bzw. geschwenkter Abdeckung;
- Fig. 5: einen schematischen Schnitt des Inhalators gemäß Fig. 2 mit geschlossener Abdeckung;
- Fig. 6: einen vergrößerten Ausschnitt der Darstellung gemäß Fig. 5;
- Fig. 7: eine perspektivische Ansicht eines Trägers des Magazins;
- Fig. 8: eine perspektivische Ansicht einer Kapselkammer;
- Fig. 9: eine perspektivische Ansicht eines Mitnehmers einer Fördereinrichtung mit einer zugeordneten Kapselkammer zum radialen Bewegen der Kapselkammer;
- Fig. 10: eine perspektivische Ansicht der Abdeckung ohne Inhalator;
- Fig. 11: eine schematische Darstellung der Zusammenwirkung der Abdeckung mit dem Mitnehmer bei radial eingeschobener Abdeckung;
- Fig. 12: eine schematische Darstellung der Zusammenwirkung der Abdeckung mit dem Mitnehmer bei radial ausgezogener Abdeckung;
- Fig. 13: eine schematische Darstellung der Zusammenwirkung der Abdeckung mit dem Mitnehmer bei leicht geschwenkter Abdeckung;
- Fig. 14: eine schematische Darstellung der Zusammenwirkung der Abdeckung mit dem Mitnehmer bei vollständig geöffneter Abdeckung;
- Fig. 15: eine schematische Darstellung der Zusammenwirkung der Abdeckung mit dem Mitnehmer bei anfänglichem Schließen der Abdeckung;
- Fig. 16: eine schematische Darstellung der Zusammenwirkung der Abdeckung mit dem Mitnehmer bei radial wieder zurückbewegtem Mitnehmer;
- Fig. 17: eine schematische Darstellung der Zusammenwirkung der Abdeckung mit dem Mitnehmer bei in die geschlossene Position zurückgeschwenkter, aber noch nicht radial eingeschobener Abdeckung;
- Fig. 18: eine schematische Ansicht des Eingriffs der Abdeckung am Magazin;
- Fig. 19: eine schematische Darstellung einer Rückdrehsicherung des Magazins durch Eingriff von Vorsprüngen des Gehäuses am Magazin bzw. Träger;
- Fig. 20: eine perspektivische Ansicht eines alternativen Mitnehmers;
- Fig. 21: einen schematischen Schnitt eines vorschlagsgemäßen Inhalators gemäß einer zweiten Ausführungsform;
- Fig. 22: eine perspektivische Ansicht eines Mitnehmers des Inhalators gemäß Fig. 21 mit zugeordneter Kapsel;
- Fig. 23: einen schematischen Schnitt des Inhalators gemäß Fig. 21 mit teilweise radial herausgezogener Abdeckung;
- Fig. 24: einen schematischen Schnitt des Inhalators gemäß Fig. 21 mit vollständig radial herausgezogener Abdeckung;
- Fig. 25: einen schematischen Schnitt eines vorschlagsgemäßen Inhalators gemäß einer dritten Ausführungsform mit geschlossener Abdeckung;
- Fig. 26: eine schematische Ansicht eines Magazins mit Kapselkammern;
- Fig. 27: einen schematischen Teilschnitt des Inhalators gemäß Fig. 25 mit vollständig radial herausgezogener Abdeckung;
- Fig. 28: eine schematische Darstellung eines Mitnehmers mit zugeordneter Kapsel;
- Fig. 29: eine schematische Darstellung der Kopplung eines Mitnehmers mit der Abdeckung;
- Fig. 30: eine schematische Darstellung zweier Steuerringe mit zugeordneten Stellschiebern und Mitnehmern;
- Fig. 31: eine perspektivische Ansicht eines Deckels;
- Fig. 32: eine schematische Darstellung des Inhalators gemäß Fig. 25 bei vollständig ausgeschwenkter Abdeckung;
- Fig. 33: eine schematische Darstellung einer Antriebseinrichtung;
- Fig. 34: eine schematische Darstellung der Antriebseinrichtung gemäß Fig. 33 im weitergedrehten Zustand des Magazins;
- Fig. 35: eine perspektivische Ansicht eines Steuerrings mit gegenüberliegenden Aussparungen und Steuerflächen; und
- Fig. 36: eine schematische Darstellung der Kopplung des Stellschiebers mit dem Steuerring.

In den Figuren werden für gleiche oder ähnliche Teile die gleichen Bezugszeichen verwendet, auch wenn eine wiederholte Beschreibung weggelassen ist. Insbesondere ergeben sich auch die gleichen oder entsprechende Vorteile und Eigenschaften. Einzelne Figuren können aus Darstellungs- oder Vereinfachungsgründen auch nicht maßstabsgerecht sein.

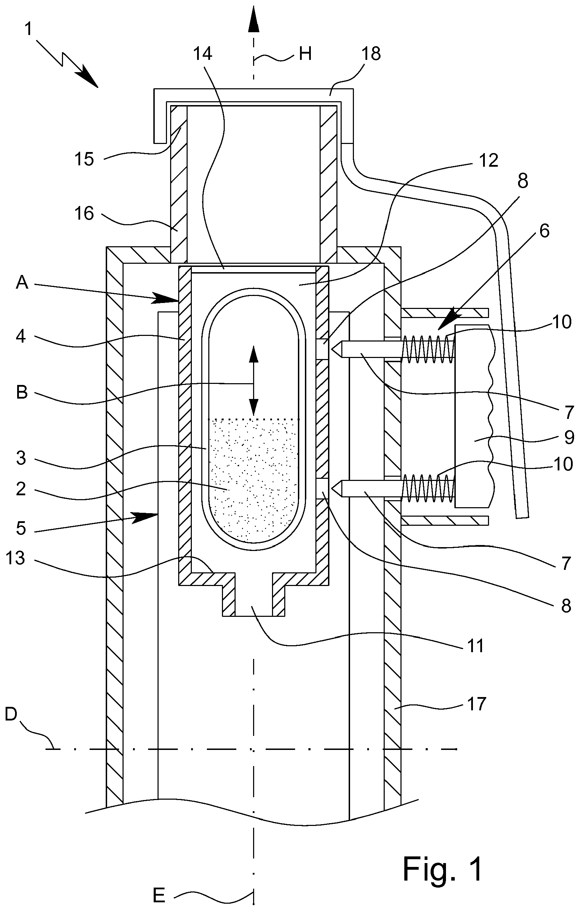

Fig. 1 zeigt in einem schematischen Schnitt den grundsätzlichen Aufbau bzw. das grundsätzliche Funktionsprinzip eines vorschlagsgemäßen Inhalators 1. Die diesbezüglichen Ausführungen gelten insbesondere entsprechend und ergänzend für alle später noch beschriebenen Ausführungsformen.

Der Inhalator 1 ist vorzugsweise tragbar ausgebildet und arbeitet insbesondere nur mechanisch.

Der Inhalator 1 dient der Ausgabe bzw. Zerstäubung, insbesondere zur Inhalation einer vorzugsweise pulverförmigen Formulierung 2 aus Kapseln 3. Die Formulierung 2 ist also vordosiert in Dosen, die in den Kapseln 3 aufgenommen sind. Bedarfsweise können die Kapseln 3 auch unterschiedliche Formulierungen 2 enthalten.

Bei der Formulierung 2 handelt es sich insbesondere um eine Formulierung im eingangs genannten Sinne.

Bei den Kapseln 3 handelt es sich insbesondere um Kapseln im eingangs genannten Sinne.

Der Inhalator 1 weist vorzugsweise mindestens eine Kapselkammer 4 auf, insbesondere wobei die Kapselkammer 4 zur Aufnahme einer (zugeordneten) Kapsel 3 ausgebildet ist. Besonders bevorzugt weist der Inhalator 1 mehrere Kapselkammern 4, insbesondere eine Kapselkammer 4 je Kapsel 3, auf. Hier sind jedoch auch andere Lösungen möglich, wie im Folgenden noch näher erläutert wird.

In dem schematischen Schnitt ist eine Kapsel 3 innerhalb einer Kapselkammer 4 des Inhalators 1 gezeigt. Die Kapsel 3 ist noch verschlossen, also noch nicht geöffnet.

Bei der Kapselkammer 4 handelt es sich insbesondere um eine Kapselkammer im eingangs genannten Sinne.

Die Kapseln 3 sind vorzugsweise länglich ausgebildet. Grundsätzlich können die Kapseln 3 jedoch auch jede sonstige geeignete Form aufweisen und beispielsweise kugelförmig ausgebildet sein.

Die Kapseln 3 können grundsätzlich aus jedem geeigneten Material hergestellt sein bzw. bestehen. Vorzugsweise wird Gelatine als Kapselmaterial verwendet. In diesem Fall kann diese im Gemisch mit anderen Zusätzen ausgewählt aus der Gruppe bestehend aus Polyethylenglycol (PEG), bevorzugt PEG 3350, Glycerol, Sorbitol, Propylenglycol, PEO-PPO-Blockcopolymeren und anderen Polyalkoholen und Polyethern zum Einsatz kommen. Besonders bevorzugt wird Gelatine im Gemisch mit PEG, bevorzugt PEG 3350, verwendet. Besonders bevorzugt enthält eine GelatineKapsel 3 PEG in einem Anteil von 1 bis 10 % (Gew.-%), bevorzugt 3 bis 8 %. Besonders bevorzugte Gelatine-Kapseln 3 enthalten PEG in einem Anteil von 4 bis 6 %, wobei ein PEG-Anteil von etwa 5 % höchstbevorzugt ist. Im Falle Gelatinehaltiger Kapselmaterialien weisen die Kapseln 3 vorzugsweise eine Tews- oder Halogentrockner-Feuchte von weniger als 12 %, besonders bevorzugt von ≤ 10 %, auf.

Werden Cellulosederivate als Kapselmaterial verwendet, so ist die Verwendung von Hydropropylmethylcellulose, Hydroxypropylcellulose, Methylcellulose, Hydroxymethylcellulose und Hydroxyethylcellulose bevorzugt. Besonders bevorzugt wird in diesem Fall Hydropropylmethylcellulose (HPMC), besonders bevorzugt HPMC 2910 als Kapselmaterial eingesetzt. Im Falle der Verwendung von Cellulosederivaten als Kapselmaterialien liegt der Grad der Tews- oder Halogentrockner-Feuchte vorzugsweise bei weniger als 8 %, besonders bevorzugt bei weniger als 5 %. Höchstbevorzugt werden Inhalationskapseln 3 aus Cellulosederivaten vor Befüllung mit einem Tiotropium-haltigen Inhalationspulver auf eine Tews- oder Halogentrockner-Feuchte von weniger als 4 %, besonders bevorzugt weniger als 2 %, getrocknet.

Die Kapseln 3 können jeweils aus einem Kapselkörper und einer Kapselkappe bestehen, wie insbesondere in der WO 00/07572 A2 offenbart. Bei diesem zweiteiligen Aufbau wird insbesondere Kunststoff als Kapselmaterial eingesetzt. Insbesondere bestehen der Kapselkörper und die Kapselkappe aus dem gleichen Material. Sie werden so miteinander verbunden, dass ein stabiler, abgeschlossener Hohlraum von definiertem Volumen gebildet wird. Besonders bevorzugt wird hierbei Kunststoff, insbesondere Polyethylen, eingesetzt.

Die Kapsel 3 kann Rastelemente aufweisen, die die Kapselkappe fest mit dem Kapselkörper verbinden.

Die Kapseln 3 und ggf. die Kapselkammern 4 (sofern mehrere Kapselkammern 4 vorhanden sind) sind vorzugsweise von einem Magazin 5 des Inhalators 1 aufgenommen. In dem schematischen Schnitt gemäß Fig. 1 ist das Magazin 5 nur schematisch angedeutet.

Das Magazin 5 ist insbesondere flach, scheibenartig oder ringartig ausgebildet. Es weist insbesondere eine Mittel- oder Hauptebene E auf.

Das Magazin 5 ist optional in den Inhalator 1 einsetzbar und/oder auswechselbar (nicht dargestellt).

Die Kapseln 3 und ggf. Kapselkammern 4 sind im oder an dem Magazin 5 vorzugsweise zumindest im Wesentlichen ringförmig angeordnet bzw. aufgenommen, bevorzugt mit zumindest im Wesentlichen radialer Ausrichtung.

Das Magazin 5 ist im Inhalator 1 vorzugsweise bewegbar bzw. drehbar, hier um eine zur Ebene E vorzugsweise senkrechte Drehachse D, insbesondere so dass das Magazin 5 von einer Kapsel 3 zur nächsten Kapsel 3 bewegbar bzw. förderbar bzw. (weiter) drehbar ist, besonders bevorzugt um die Kapseln 3 einzelweise in eine Ausgabeposition A, wie beispielhaft in Fig. 1 dargestellt, zu bringen.

Wie bereits erwähnt, enthält jede Kapsel 3 vorzugsweise eine Dosis der Formulierung 2. Da das Magazin 5 eine Vielzahl von Kapseln 3 und eine entsprechende Anzahl von Dosen enthält, kann der Inhalator 1 beispielsweise für eine Woche oder mehrere Wochen oder sogar für einen Monat die Versorgung eines Benutzers bzw. Patienten mit der Formulierung 2, also einem Medikament oder dergleichen, sicherstellen.

Die Kapseln 3 sind vorzugsweise einzelweise im Inhalator 1 öffenbar. Zum Öffnen weist der Inhalator 1 eine Öffnungseinrichtung 6 auf, die der Kapselkammer 4 bzw. Kapsel 3 bzw. Ausgabeposition A zugeordnet ist. Bedarfsweise können mehrere Öffnungseinrichtungen 6 vorgesehen sein, die jeweils einer Kapselkammer 4 bzw. Kapsel 3 zugeordnet sind. Vorzugsweise ist jedoch nur eine gemeinsame Öffnungseinrichtung 6 vorgesehen.

Vorzugsweise erfolgt das Öffnen der Kapsel 3 jeweils durch Anstechen. Hierzu weist die Öffnungseinrichtung 6 vorzugsweise mindestens ein Anstechelement, wie eine Nadel 7, beim Darstellungsbeispiel zwei Nadeln 7, auf.

Die Kapselkammer 4 weist vorzugsweise entsprechende Anstechöffnungen, hier Nadelöffnungen 8, für die Anstechelemente bzw. Nadeln 7 auf. Vorzugsweise sind die Anstechelemente in die Anstechöffnungen vorgerückt und verschließen diese. Diese Stellung nimmt die Öffnungseinrichtung 6 nach dem Öffnen einer Kapsel 3 während der Inhalation ein, um die Anstechöffnungen der Kapselkammer 4 zumindest weitestgehend abzudichten. Jedoch sind auch andere Arten des Verschlusses bzw. der Abdichtung möglich, insbesondere von innen und/oder von außen bzw. durch sich selbsttätig oder automatisch schließende Anstechöffnungen oder Verschlusseinrichtungen.

Vorzugsweise erfolgt ein seitliches Öffnen oder Anstechen der jeweiligen Kapsel 3. Hierzu ist die vorzugsweise längliche Kapsel 3 längsseitig bzw. seitlich und/oder quer zur Hauptströmungsrichtung oder Längsachse der Kapselkammer 4 anstechbar und dadurch öffenbar.

Besonders bevorzugt wird die Kapsel 3 beim Öffnen im Bereich ihrer beiden Enden und/oder seitlich angestochen.

Das Anstechen bzw. Öffnen von Kapseln 3 erfolgt vorzugsweise wie in der EP 0 147 755 A2 beschrieben.

Die Öffnungseinrichtung 6 weist vorzugsweise ein Betätigungselement 9 auf, das insbesondere manuell betätigbar ist. Vorzugsweise ist durch Betätigung oder Niederdrücken oder Eindrücken des Betätigungselements 9 die jeweilige Kapsel 3 anstechbar.

Beim Darstellungsbeispiel sind die Anstechelemente bzw. Nadeln 7 vorzugsweise fest mit dem Betätigungselement 9 verbunden. Jedoch sind auch andere konstruktive Lösungen möglich.

Vorzugsweise ist die Öffnungseinrichtung 6 bzw. das Betätigungselement 9 gegen die Kraft eines Rückstellelements, wie mindestens einer Feder 10, betätigbar. Mittels des Rückstellelements bzw. der Feder 10 nehmen die Anstechelemente bzw. Nadeln 7 nach dem Betätigen bzw. Loslassen des Betätigungselements 9 vorzugsweise wieder ihre zurückgezogene Position der Ausgangsstellung, wie in Fig. 1 angedeutet, ein. Jedoch sind auch andere konstruktive Lösungen möglich.

Der Inhalator 1 kann mehrere Kapselkammern 4 aufweisen, in die jeweils eine Kapsel 3 aufgenommen ist und die jeweils insbesondere nur einmal verwendet werden. Jedoch kann, wie bereits erwähnt, der Inhalator 1, auch nur eine Kapselkammer 4 aufweisen, in die die Kapseln 3 nacheinander für die Ausgabe bzw. Entleerung aufgenommen werden. Hier erfolgt dann also eine Mehrfachverwendung der Kapselkammer 4.

Die Kapselkammer 4 ist wahlweise ein- oder mehrteilig ausgebildet. Dafür kann die Kapselkammer 4 auch aus unterschiedlichen Materialien und/oder durch ZweiKomponenten-Spritzguss aufgebaut bzw. hergestellt sein.

Die Kapselkammer 4 weist vorzugsweise einen Einlass 11 und einen Auslass 12 auf, die sich insbesondere axial bzw. stirnseitig an einen insbesondere länglichen bzw. zylindrischen bzw. zentralen Bewegungs- bzw. Aufnahmebereich der Kapselkammer 4 für die Kapsel 3 anschließen.

Besonders bevorzugt ist der Einlass 11 in seinem Durchmesser gegenüber dem Aufnahmebereich für die Kapsel 3 verringert, so dass vorzugsweise eine Ringschulter 13 oder dergleichen gebildet wird, die einen Anschlag oder eine Wegbegrenzung für die Kapsel 3 bildet.

Dem Auslass 12 ist vorzugsweise ein Sicherungselement 14 zugeordnet, das beispielsweise gitterartig oder stegartig ausgebildet ist und/oder ein Herausbewegen der Kapsel 3 aus dem Aufnahmebereich bzw. aus der Kapselkammer 4 verhindert.

Vorzugsweise entsprechen die geometrischen Verhältnisse zumindest im Wesentlichen den Angaben in der EP 0 147 755 A2.

Bei der Inhalation bzw. Ausgabe strömt Luft oder sonstiges Gas durch den Einlass 11 in die Kapselkammer 4, durch diese bzw. deren Aufnahmebereich hindurch und aus dem vorzugsweise gegenüberliegenden Auslass 12 wieder hinaus. Dieser Luft- bzw. Gasstrom kann durch das Einatmen beim Inhalieren und/oder durch einen dem Inhalator 1 zugeordneten Druckerzeuger, wie eine Luftpumpe, einem Druckgasreservoir oder dergleichen erzeugt werden.

Der genannte Luftstrom durch die Kapselkammer 4 bewirkt, insbesondere durch den Bernoulli-Effekt, dass die Kapsel 3 in der Kapselkammer 4 vibriert oder schwingt bzw. sich insbesondere axial hin- und her bewegt, wie durch Pfeil B in Fig. 1 angedeutet. Diese Bewegung oder Schwingung B bewirkt oder unterstützt den Austrag der Formulierung 2 aus der geöffneten bzw. angestochenen Kapsel 3 in den Luftstrom - insbesondere in Form von sehr feinen Partikeln - und ein Dispergieren der Formulierung 2 in den Luftstrom, mit dem die Formulierung 2 schließlich über den Auslass 12 und besonders bevorzugt ein sich anschließendes Mundstück 15 des Inhalators 1 an einen nicht darstellten Benutzer bzw. Patienten ausgegeben wird.

Die Hauptausgaberichtung H der dispergierten Formulierung 2 mittels des Luftstroms bzw. des Inhalators 1 ist in Fig. 1 durch einen entsprechenden Pfeil angedeutet. Die Ausgabe ist natürlich nur bei geöffneter Abdeckung 18 und nach Öffnen der jeweiligen Kapsel 3 möglich.

Das Ausgeben bzw. Dispergieren der Formulierung 2 erfolgt insbesondere wie in der EP 0 147 755 A2 beschrieben. Jedoch kann die Formulierung 2 grundsätzlich auch auf jede sonstige geeignete Weise aus der Kapsel 3 ausgetragen werden, beispielsweise durch Rotation der Kapsel 3 quer zu ihrer Längsachse oder dergleichen.

Der Inhalator 1 weist also vorzugsweise ein Mundstück 15 oder eine sonstige Einrichtung zur Ausgabe der in dem Luft- bzw. Gasstrom dispergierten Formulierung 2 auf.

Beim Darstellungsbeispiel schließt sich das Mundstück 15, insbesondere mit einem Verbindungsabschnitt 16, an die Kapselkammer 4 bzw. den Auslass 12 an.

Das Sicherungselement 14 kann je nach Bedarf und Konstruktion dem Magazin 5 bzw. der Kapselkammer 4 zugeordnet und/oder daran angeordnet sein, bedarfsweise aber auch vom Inhalator 1 bzw. Mundstück 15 oder Verbindungsabschnitt 16 gebildet oder gehalten sein.

Der Inhalator 1 weist vorzugsweise ein Gehäuse 17 auf. Hierbei handelt es sich insbesondere um ein Außengehäuse.

Vorzugsweise ist das Mundstück 15 am Gehäuse 17 insbesondere feststehend oder unlösbar angeordnet bzw. gebildet. Jedoch sind noch andere konstruktive Lösungen möglich.

Der Inhalator 1 bzw. das Gehäuse 17 ist vorzugsweise zumindest im Wesentlichen scheibenartig und/oder flach ausgebildet.

Das Mundstück 15 ist vorzugsweise peripher am Inhalator 1 bzw. Gehäuse 17 angeordnet.

Das Mundstück 15 bzw. die Hauptausgaberichtung H des Inhalators 1 bzw. die Bewegung der Kapsel 3 bei Inhalation verläuft vorzugsweise zumindest im Wesentlichen radial zu der Drehachse D des Magazins 5.

Der Inhalator 1 weist vorzugsweise eine dem Mundstück 15 zugeordnete Abdeckung 18 zum wahlweisen Öffnen und Schließen des Mundstücks 15 auf.

Bei geöffneter Abdeckung 18 ist das Mundstück 15 zur Inhalation freigegeben. Bei geschlossener Abdeckung 18, wie in Figur 1 dargestellt, ist das Mundstück 15 vorzugsweise abgedeckt. Eine Inhalation ist also nicht möglich.

In der geschlossenen Position kann die Abdeckung 18 optional, wie in Fig. 1 angedeutet, die Öffnungseinrichtung 6 bzw. deren Betätigungselement 9 mit abdecken oder auf sonstige Weise blockieren, um ein (unerwünschtes) Öffnen der Kapsel 3 in diesem Zustand durch einen Benutzer auszuschließen bzw. zu verhindern.

Die Abdeckung 18 ist vorzugsweise (um eine zur Drehachse D koaxiale oder dazu parallele, beispielsweise außen am Gehäuse 17 liegende, Schwenkachse) schwenkbar oder drehbar am Inhalator 1 bzw. Gehäuse 17 gehalten oder gelagert. Alternativ oder zusätzlich ist die Abdeckung 18 vorzugsweise auch linear bzw. radial bewegbar, wie im Folgenden noch näher erläutert wird.

Die Abdeckung 18 ist vorzugsweise form- und/oder kraftschlüssig, insbesondere untrennbar, mit dem Gehäuse 17 verbunden oder am bzw. im Gehäuse 17 gelagert, beispielsweise durch Einrasten.

Nach der Entleerung einer Kapsel 3 bzw. nach der Inhalation der Formulierung 2 einer Kapsel 3, wird die nächste Kapsel 3 in die Ausgangsposition A gebracht bzw. bewegt. Dies erfolgt insbesondere durch Weiterbewegen bzw. Drehen des Magazins 5.

Wenn nur eine Kapselkammer 4 für mehrere oder alle Kapseln 3 vorgesehen ist, wird die entleerte Kapsel 3 zunächst aus der Kapselkammer 4 herausbewegt, beispielsweise durch einlassseitiges Öffnen der Kapselkammer 4. Anschließend kann die nächste Kapsel 3 in die Kapselkammer 4 bewegt bzw. eingeführt und die Kapselkammer 4 wieder geschlossen werden. Diese neue Kapsel 3 wird dann insbesondere erst kurz vor der nächsten Inhalation geöffnet bzw. angestochen.

Ein Öffnen und Schließen der Kapselkammer 4 entfällt hingegen, wenn jede Kapsel 3 in einer separaten Kapselkammer 4 aufgenommen ist, also nur die Kapselkammern 4 zusammen mit den Kapseln 3 jeweils einzelweise in die Ausgabeposition A gebracht bzw. bewegt werden müssen.

Zur Benutzung öffnet ein nicht dargestellter Benutzer bzw. Patient den Inhalator 1 bzw. die Abdeckung 18. Hierdurch wird das Mundstück 15 freigegeben. Dann erfolgt eine Betätigung der Öffnungseinrichtung 6, also ein Anstechen der in der Ausgabeposition A befindlichen Kapsel 3.

Die Ausgabeposition A bezeichnet insbesondere eine Position der Kapsel 3 bzw. Kapselkammer 4 benachbart zu dem Mundstück 15 bzw. dessen Verbindungsabschnitt 16.

In der Ausgabeposition A liegt die Kapsel 3 bzw. deren Kapselkammer 4 insbesondere in Verlängerung zu dem Verbindungsabschnitt 16 bzw. Mundstück 15 und/oder mit ihrer Längsachse ausgerichtet zu und/oder in Verlängerung der Hauptausgaberichtung H und/oder in radialer Richtung, insbesondere bezogen auf die Drehachse D des Magazins 5.

Nach dem Öffnen bzw. Anstechen der Kapsel 3 kann die Inhalation erfolgen. Anschließend wird der Inhalator 1 wieder geschlossen. Weiter wird dann die nächste Kapsel 3 bzw. Kapselkammer 4 in die Ausgabeposition A bewegt, insbesondere durch Weiterbewegen bzw. Weiterdrehen des Magazins 5 zur nächsten Kapsel 3 bzw. Kapselkammer 4.

Nachfolgend werden verschiedene Ausführungsformen des vorschlagsgemäßen Inhalators 1 und Magazins 5 näher erläutert, wobei die vorherigen Ausführungen und Erläuterungen insbesondere entsprechend oder ergänzend gelten, auch wenn eine wiederholte Beschreibung weggelassen ist.

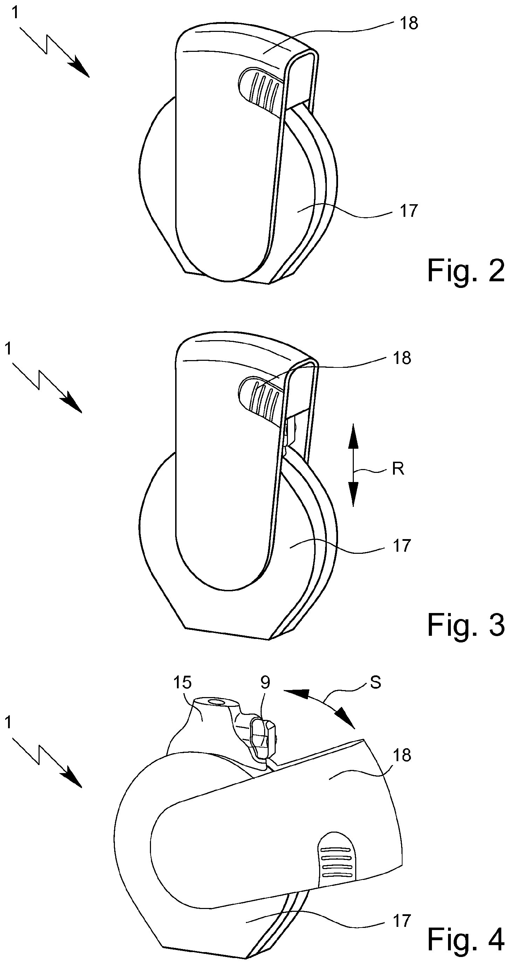

Fig. 2 bis 4 zeigen perspektivische Ansichten einer ersten Ausführungsform des vorschlagsgemäßen Inhalators 1 in verschiedenen Zuständen.

In Fig. 2 ist der Inhalator 1 bzw. dessen Abdeckung 18 geschlossen. Dies ist die Ausgangsstellung bzw. geschlossene Stellung.

In Fig. 3 ist die Abdeckung 18 radial angehoben bzw. ausgezogen. Diese Radialbewegung R bildet einen ersten Teil der Öffnungsbewegung der Abdeckung 18. Insbesondere ist ausgehend von dem in Fig. 2 dargestellten, geschlossenen Zustand zunächst nur die radiale Bewegung der Abdeckung 18 in die radial ausgezogene bzw. vom Mundstück 15 mehr abgerückte Stellung, wie in Fig. 3 dargestellt, möglich. Diese Stellung wird nachfolgend kurz auch als "erste Öffnungsstellung" der Abdeckung 18 bezeichnet.

Die Radialbewegung R verläuft insbesondere in die Hauptausgaberichtung H, so dass generell anstelle der Radialbewegung R auch eine Bewegung in die Hauptausgaberichtung H gemeint sein kann.

Ausgehend von der ersten Öffnungsstellung wird die Abdeckung 18 geschwenkt, um das Mundstück 15 freizugeben, also zu öffnen, bzw. den Inhalator 1 vollständig zu öffnen. Diese geöffnete Stellung wird nachfolgend kurz auch als "zweite Öffnungsstellung" der Abdeckung 18 bezeichnet.

Fig. 4 zeigt diese Stellung. Die Schwenkbewegung S von der ersten Öffnungsstellung in die zweite Öffnungsstellung ist durch einen Pfeil in Fig. 4 angedeutet.

In der zweiten Öffnungsstellung ist das Mundstück 15 (vollständig) freigegeben. Weiter gibt die Abdeckung 18 in der zweiten Öffnungsstellung die Öffnungseinrichtung 6 bzw. deren Betätigungselement 9 frei, deckt diese also nicht mehr ab, so dass eine manuelle Betätigung nun möglich ist.

Die Schwenkbewegung S stellt einen zweiten Teil der Öffnungsbewegung dar.

Die verschiedenen Teile der Öffnungsbewegung dienen vorzugsweise unterschiedlichen Zwecken bzw. Funktionen.

Insbesondere dient die Radialbewegung R bzw. der erste Teil der Öffnungsbewegung einer insbesondere radialen Bewegung oder Förderung der Kapsel 3 und ggf. auch einer zugeordneten Kapselkammer 4 aus dem Magazin 5 in die Ausgabeposition A.

Bei der ersten Ausführungsform liegt die Ausgabeposition A insbesondere in dem Mundstück 15 bzw. Verbindungsabschnitt 16 und/oder vorzugsweise radial außerhalb des Magazins 5.

Die Schwenkbewegung S, insbesondere von der zweiten bzw. ganz geöffneten Öffnungsstellung (Fig. 4) in die Zwischenstellung bzw. erste Öffnungsstellung (Fig. 3), also das Zuschwenken der Abdeckung 18, dient vorzugsweise einer Weiterförderung bzw. einem Weiterdrehen oder Weitertakten des Magazins 5.

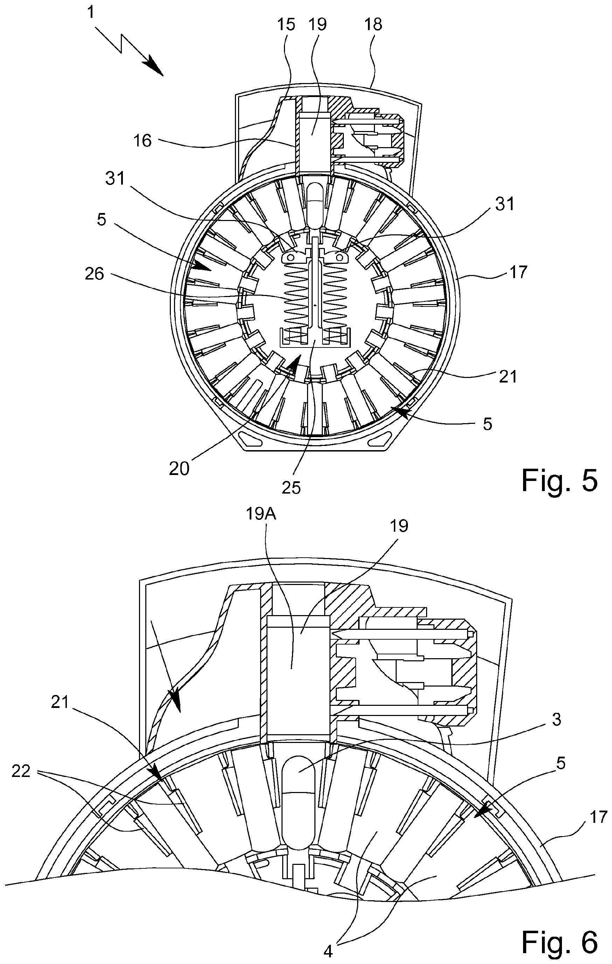

Fig. 5 zeigt in einem schematischen Schnitt des Inhalators 1 in seiner Hauptebene E den bevorzugten Aufbau des Magazins 5 und Inhalators 1. Der Inhalator 1 befindet sich in seiner Ausgangsposition.

Vorzugsweise weist der Inhalator 1 eine Aufnahmekammer 19 zur einzelweisen Aufnahme einer Kapsel 3 mit Kapselkammer 4 auf. Diese Aufnahmekammer 19 wird insbesondere von dem Mundstück 15 bzw. dessen Verbindungsabschnitt 16 gebildet und/oder definiert die Ausgabeposition A.

Bei der Darstellung gemäß Fig. 5 ist die Aufnahmekammer 19 leer, die Ausgabeposition A also (noch) nicht besetzt. Die Abdeckung 18 bzw. der Inhalator 1 befindet sich in der geschlossenen Position bzw. in der Ausgangsposition.

Die Kapselkammern 4 sind einzelweise jeweils zusammen mit der jeweils darin befindlichen Kapsel 3 aus dem Magazin 5 radial in die Aufnahmekammer 19 bzw. Ausgangsposition A bewegbar oder förderbar. Nach dem Öffnen der jeweiligen Kapsel 3 und Ausgabe der Formulierung 2 der geöffneten Kapsel 3 wird die Kapselkammer 4 wieder in das Magazin 5 zurückbewegt bzw. zurückgezogen.

Zur insbesondere radialen Bewegung der Kapselkammern 4 aus dem Magazin 5 in die Ausgabeposition A und/oder umgekehrt, weist der Inhalator 1 vorzugsweise eine Fördereinrichtung 20 auf. Diese ist vorzugsweise zumindest im Wesentlichen innerhalb des Magazins 5 bzw. in der Mitte des Magazins 5 und/oder Inhalators 1 angeordnet.

Fig. 6 zeigt in einem vergrößerten Ausschnitt des Inhalators gemäß Fig. 5 eine ersten Ausführungsform des vorschlagsgemäßen Magazins 5 mit angrenzender Aufnahmekammer 19.

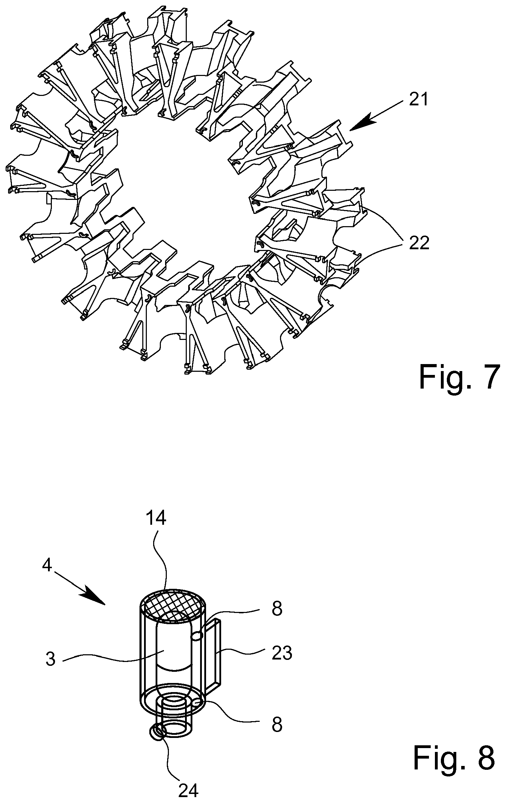

Die Kapselkammern 4 sind in dem Magazin 5 radial beweglich bzw. entnehmbar aufgenommen. Das Magazin 5 weist hierzu vorzugsweise einen in Fig. 5 bis Fig. 7 schematisch dargestellten Träger 21 auf, insbesondere wobei der Träger 21 die Kapselkammern 4 in gewünschter Weise, insbesondere klemmend und/oder rastend, hält. Vorzugsweise weist der Träger 21 hierzu entsprechende Haltearme oder -abschnitte 22 auf, wie insbesondere Fig. 6 und Fig. 7 veranschaulichen.

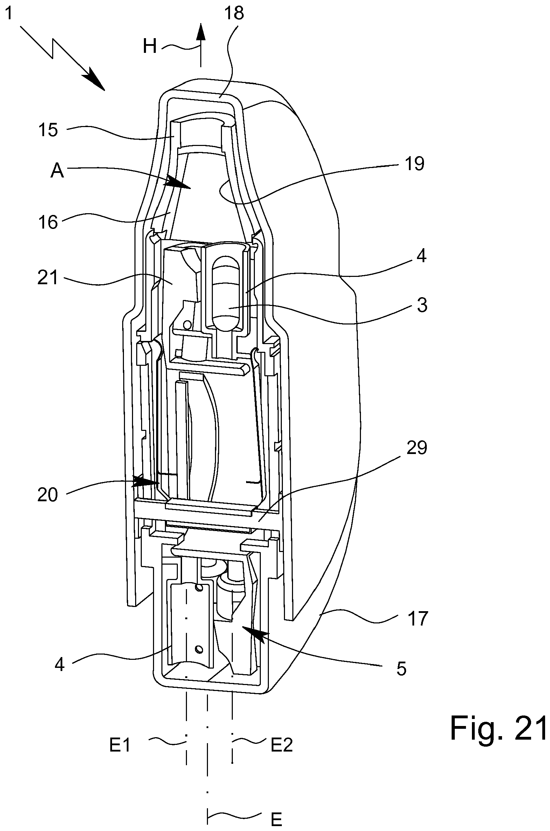

Die Kapselkammern 4 sind in dem Magazin 5 vorzugsweise in verschiedenen Ebenen, insbesondere in zwei Ebenen E1 und E2, aufgenommen. Die beiden Kapselkammerebenen E1 und E2 sind zueinander in axialer Richtung (bezüglich der Drehachse D des Magazins 5) beabstandet oder versetzt und/oder symmetrisch auf beiden Seiten der Hauptebene E des Magazins 5 angeordnet. Die Kapselkammerebenen E1 und E2 sind in Fig. 21, auf die später näher eingegangen wird, schematisch angedeutet.

Die beiden Ringanordnungen von Kapselkammern 4 in den beiden Ebenen E1 und E2 sind vorzugsweise in Umfangsrichtung derart zueinander versetzt bzw. verdreht, dass eine Kapselkammer 4 einer der Ebenen E1 und E2 immer zwischen zwei Kapselkammern 4 der anderen Ebene E1 bzw. E2 liegt. Insbesondere können dann die beiden Ringanordnungen von Kapselkammern 4 bzw. die beiden Ebenen E1 und E2 etwas näher axial zusammengerückt werden. So ist eine besonders kompakte Ausbildung des Magazins 5 möglich bzw. können besonders viele Kapselkammern 4 im Magazin 5 angeordnet werden.

Das Mundstück 15 bzw. der Verbindungsabschnitt 16 bzw. die Aufnahmekammer 19 bzw. die Längsachse dessen bzw. dieser bzw. die Ausgabeposition A ist vorzugsweise zwischen den beiden Ebenen E1 bzw. E2 und/oder versetzt zu der Ebene E1 und/oder der Ebene E2 angeordnet, insbesondere derart, dass die Kapseln 3 bzw. Kapselkammern 4 der Ebene E1 und/oder der Ebene E2 schräg aus dem Magazin 5 in die Aufnahmekammer 19 eingeführt werden.

Vorzugsweise weist der Inhalator 1, insbesondere das Mundstück 15 bzw. der Verbindungsabschnitt 16 bzw. die Aufnahmekammer 19, mindestens eine Einführschräge 19A auf oder bildet eine solche, vorzugsweise wobei die Kapseln 3 bzw. die Kapselkammern 4 der Ebene E1 und/oder der Ebene E2 mittels der Einführschräge(n) 19A aus dem Magazin 5 in die Aufnahmekammer 19 bzw. aus der Ebene E1 bzw. E2 in die - vorzugsweise zu der Ebene E1 und/oder E2 versetzte - Ausgabeposition A bringbar bzw. bewegbar und/oder beim Einführen in die Aufnahmekammer 19 zentrierbar sind.

Besonders bevorzugt verjüngt sich die Aufnahmekammer 19 von innen nach außen bzw. in Richtung des Mundstücks 15, vorzugsweise mittels der Einführschräge(n) 19A, insbesondere derart, dass sich die Kapseln 3 bzw. Kapselkammern 4 beim Einführen in die Aufnahmekammer 19 selbsttätig zentrieren bzw. die - vorzugsweise zu der Ebene E1 und/oder E2 versetzte - Ausgabeposition A einnehmen.

Insbesondere sind sowohl die Kapseln 3 bzw. Kapselkammern 4 der Ebene E1 als auch der Ebene E2 in die gleiche Ausgabeposition A bringbar bzw. bewegbar, vorzugsweise mittels der Einführschräge(n) 19A.

Vorzugsweise werden sowohl die Kapseln 3 bzw. Kapselkammern 4 der Ebene E1 als auch der Ebene E2 bzw. alle Kapseln 3 bzw. Kapselkammern 4 mittels der Einführschräge(n) ausgerichtet bzw. schräg in die Ausgabeposition A gebracht. Hier sind jedoch auch andere Lösungen möglich, insbesondere bei denen lediglich die Kapseln 3 bzw. Kapselkammern 4 einer der Ebenen E1, E2 schräg in die Aufnahmekammer 19 eingeführt werden müssen bzw. die Aufnahmekammer 19 bzw. deren Längsachse bzw. die Ausgabeposition A in einer der Ebenen E1, E2 liegt.

Beim Darstellungsbeispiel weist das Magazin 5 vorzugsweise 15 Kapselkammern 4 und damit auch Kapseln 3 in jeder Ebene E1 bzw. E2 und damit insgesamt 30 Kapselkammern 4 und Kapseln 3 auf.

Fig. 8 zeigt eine perspektivische Ansicht einer Kapselkammer 4 in einer bevorzugten Ausgestaltung. Die Kapselkammer 4 weist zusätzlich zu den seitlichen Nadelöffnungen 8 vorzugsweise das Sicherungselement 14 zur Sicherung der in der Kapselkammer 4 angeordneten Kapsel 3 gegen Herausfallen auf.

Die Kapselkammer 4 weist vorzugsweise eine Führung 23 zur Festlegung der Drehlage der Kapselkammer 4 im Magazin 5 und/oder in der Ausgabeposition A bzw. Aufnahmekammer 19 auf. Die Führung 23 gestattet das Längsverschieben bzw. radiale Bewegen der Kapselkammer 4, wobei jedoch eine definierte Drehlage zumindest in der Ausgabeposition A eingenommen wird, um eine gewünschte Drehlage der Nadelöffnungen 8 und damit ein sicheres definiertes Eingreifen der Nadeln 7 in die Nadelöffnungen 8 zum Öffnen der jeweiligen Kapsel 3 sicherzustellen. Grundsätzlich ist es jedoch auch möglich, dass die Nadeln 7 die Kapselkammer 4 anstechen, ohne dass definierte Nadelöffnungen 8 vorhanden sind; in diesem Fall durchstechen die Nadeln 7 die Kapselkammer 4 an einer beliebigen Stelle. Dann kann die Kapselkammer 4 auch in ihrer Drehlage undefiniert und beispielsweise vollständig rotationssymmetrisch ausgebildet sein und die Führung 23 entfallen.

Beim Darstellungsbeispiel ist die Führung 23 insbesondere durch mindestens einen entsprechenden Abschnitt an der Kapselkammer 4 gebildet, die bzw. der sich vorzugsweise entlang der Längsachse der Kapselkammer 4 bzw. radialen Bewegungsrichtung der Kapselkammer 4 erstreckt und/oder seitlich bzw. quer dazu außen an der Kapselkammer 4 hervorragt oder ausgenommen ist und/oder beispielsweise rippenartig oder nutartig ausgebildet ist. Im Magazin 5 bzw. Träger 21 bzw. in der Aufnahmekammer 19 ist dann eine vorzugsweise zumindest im Wesentlichen komplementäre Eingriffsmöglichkeit für die Führung 23 bzw. des an der Kapselkammer 4 angeordneten Abschnitts vorgesehen. Jedoch sind auch andere konstruktive Lösungen möglich.

Dementsprechend sind die Kapselkammern 4 mit definierter Drehlage in die Ausgabeposition A und/oder radial bewegbar.

Die Kapselkammer 4 weist vorzugsweise einen Angriffsabschnitt 24 auf, an dem die Fördereinrichtung 20 angreifen kann. Der Angriffsabschnitt 24 ist vorzugsweise zapfenartig und/oder pilzkopfartig ausgebildet.

Der Angriffsabschnitt 24 ist vorzugsweise an jeder Kapselkammer 4 außen und/oder im Bereich des Einlasses 11 gebildet.

Der Angriffsabschnitt 24 erstreckt sich vorzugsweise quer zur Längserstreckung oder Hauptachse der Kapselkammer 4 und/oder in Umfangsrichtung oder axial im Magazin 5 und/oder quer bzw. senkrecht zur Radialbewegung R.

Die Fördereinrichtung 20 weist vorzugsweise einen Mitnehmer 25 und/oder eine Rückstellfeder 26 auf, wie in Fig. 5 schematisch angedeutet.

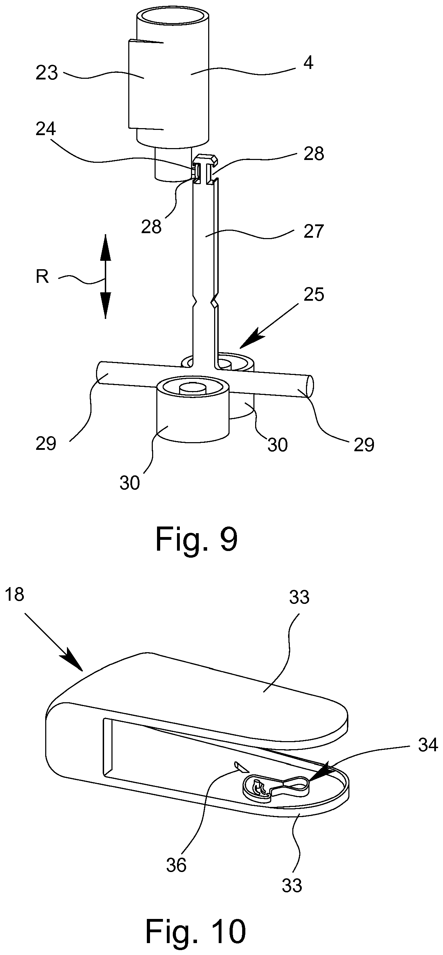

Fig. 9 zeigt in einer perspektivischen Ansicht den Mitnehmer 25 und eine zugeordnete Kapselkammer 4, insbesondere wie der Mitnehmer 25 mittels eines Angriffselements 27 an der Kapselkammer 4 bzw. dessen Angriffsabschnitt 24 angreift, um die Kapselkammer 4 radial bewegen zu können.

Das Angriffselement 27 ist insbesondere armartig ausgebildet und/oder mit einer Ausnehmung 28 zur Aufnahme oder zum Eingriff des Angriffsabschnitts 24 der jeweiligen Kapselkammer 4 ausgebildet.

Besonders bevorzugt ist die Ausnehmung 28 beim Darstellungsbeispiel in Umfangsrichtung bzw. Drehrichtung des Magazins 5 offen, so dass sich die jeweilige Kapselkammer 4 mit ihrem Angriffsabschnitt 24 beim Weiterdrehen des Magazins 5 in die Ausnehmung 28 hineinbewegen kann, um den vorzugsweise formschlüssigen Eingriff in Radialrichtung, also in Förderrichtung der Fördereinrichtung 20 bzw. in Richtung der radialen Bewegung des Mitnehmers 25, herstellen zu können.

Der Mitnehmer 25 bzw. das Angriffselement 27 weist vorzugsweise auf entgegengesetzten Seiten jeweils eine Ausnehmung 28 auf, so dass abwechselnd in die eine und in die andere Ausnehmung 28 abwechselnd Kapselkammern 4 aus den verschiedenen Ebenen E1 und E2 eingreifen können.

Der Mitnehmer 25 erstreckt sich mit seinem Angriffselement 27 vorzugsweise in der Hauptebene E des Magazins 5 zwischen die beiden Ringanordnungen von Kapselkammern 4 in den beiden Ebenen E1 und E2, insbesondere um abwechselnd mit den Kapselkammern 4 in den verschiedenen Ebenen E1, E2 in Eingriff treten zu können.

Der Mitnehmer 25 weist vorzugsweise ein Eingriffselement 29 auf, das sich insbesondere parallel zur Drehachse D bzw. quer oder senkrecht zur Hauptebene E bzw. Radialbewegung R und/oder zum Angriffselement 27 erstreckt. Das Eingriffselement 29 ragt vorzugsweise in entgegengesetzten Richtungen vom Mitnehmer 25 ab oder vor.

Das Eingriffselement 29 ist vorzugsweise armartig, stabartig oder bolzenartig ausgebildet.

Der Mitnehmer 25 weist vorzugsweise mindestens eine Federaufnahme 30, beim Darstellungsbeispiel zwei Federaufnahmen 30, zur Aufnahme bzw. Lagerung der Rückstellfeder 26, hier insbesondere von zwei Rückstellfedern 26, wie in Fig. 5 angedeutet, auf.

Die Rückstellfedern 26 sind mit ihren anderen Enden vorzugsweise an Lagerabschnitten 31 des Gehäuses 17 widergelagert. Die Rückstellfedern 26 und Lagerabschnitte 31 sind vorzugsweise innerhalb der von dem Magazin 5 gebildeten Ringanordnung bzw. innerhalb des Magazins 5 und/oder zumindest im Wesentlichen in der Hauptebene E angeordnet.

Die Rückstellfedern 26 spannen oder bewegen den Mitnehmer 25 in die in Fig. 5 gezeigte Ausgangsposition, also den Mitnehmer 25 in seine radial zurückgezogene Position.

Der Mitnehmer 25 ist längs bzw. radial verschiebbar bzw. in Richtung der Hauptausgaberichtung H im Inhalator 1 bzw. vom Gehäuse 17 verschiebbar oder beweglich geführt.

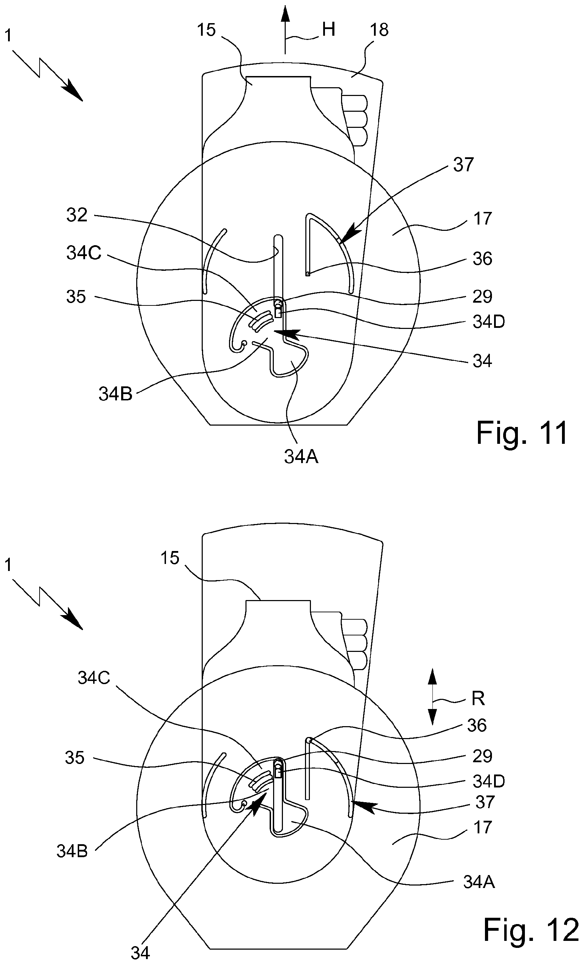

Beim Darstellungsbeispiel durchgreift der Mitnehmer 25 insbesondere mit seinem Eingriffselement 29 einen Führungsschlitz 32 im Gehäuse 17, wie in Fig. 11 dargestellt, besonders bevorzugt entsprechende Führungsschlitze 32 auf entgegengesetzten Flachseiten des Magazins 5 bzw. Gehäuses 17 mit den in die entgegengesetzte Richtung ragenden Enden seines Eingriffselements 29, so dass der Mitnehmer 25 entsprechend radial verschieblich geführt ist.

Der Mitnehmer 25 bzw. dessen Angriffselement 27 kann auch verschieblich geführt sein, vorzugsweise zwischen den beiden Lagerabschnitten 31 und sonstigen Führungsabschnitten des Gehäuses 17 oder dergleichen.

Fig. 10 zeigt in einer perspektivischen Ansicht die Abdeckung 18. Diese umgreift den Inhalator 1 bzw. das Gehäuse 17 vorzugsweise zumindest im Wesentlichen U-förmig.

Die sich vorzugsweise auf beide Flachseiten des Gehäuses 17 erstreckenden Schenkel 33 der Abdeckung 18 sind vorzugsweise flach- und/oder lappenartig ausgebildet und erstrecken sich bis über die Zentralachse bzw. Drehachse D hinaus, besonders bevorzugt im Wesentlichen bis in die Nähe der dem Mundstück 15 gegenüberliegenden Umfangsseite bzw. des Endes des Gehäuses 17 in der geschlossenen Stellung, wie in Fig. 11 angedeutet.

Die Abdeckung 18 bzw. vorzugsweise jeder Schenkel 33 weist insbesondere (vorzugsweise an der Innenseite) eine Steuereinrichtung bzw. Steuerkulisse 34 und/oder ein Steuerelement 35 für den Mitnehmer 25 bzw. ein freies Ende des Eingriffselements 29 zur Steuerung der Radialbewegung R bzw. der Fördereinrichtung 20 bzw. des Mitnehmers 25 auf.

Das Steuerelement 35 ist vorzugsweise bewegbar bzw. verschiebbar in der Abdeckung 18, insbesondere in der Steuerkulisse 34, gelagert, vorzugsweise um das Eingriffselement 29 in gewünschter Weise zu führen, wie im Folgenden noch näher erläutert wird.

Beim Darstellungsbeispiel sind die beiden Schenkel 33 vorzugsweise symmetrisch ausgebildet bzw. erfolgt eine beidseitige Führung und Steuerung des Mitnehmers 25.

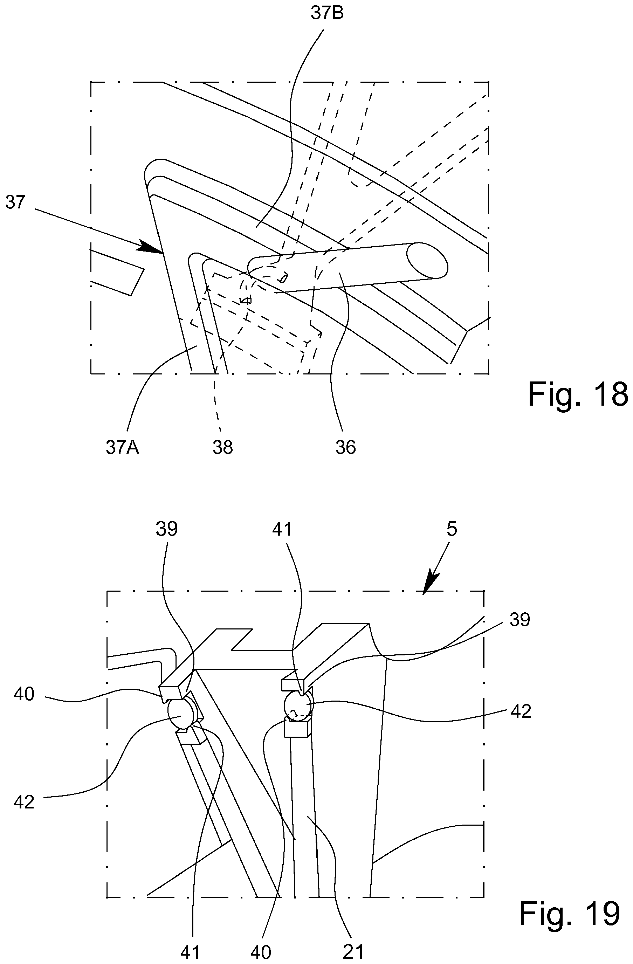

Die Abdeckung 18 bzw. mindestens einer der Schenkel 33 oder jeder Schenkel 33 weist ferner vorzugsweise ein insbesondere armartiges Antriebselement 36 zum Antreiben bzw. Drehen des Magazins 5 auf, wie in Fig. 10 oder 18 angedeutet. Das mindestens eine Antriebselement 36 ragt insbesondere armartig und/oder nach innen vor und durchgreift eine zugeordnete Führungskulisse 37, die im Gehäuse 17 (benachbart zum Führungsschlitz 32) gebildet ist, wie insbesondere in Fig. 11 angedeutet.

Nachfolgend wird zunächst die bevorzugt Steuerung der Radialbewegung R durch Bewegung der Abdeckung 18 anhand der Fig. 11 bis 17 näher erläutert. Diese Figuren zeigen den Inhalator 1 in einer sehr schematischen, teilweise durchsichtigen Darstellung zur Veranschaulichung der Funktionen bei verschiedenen Stellungen der Abdeckung 18.

Fig. 11 zeigt die Ausgangsstellung. Die Abdeckung 18 befindet sich im geschlossenen Zustand, ist also radial eingeschoben und verschließt das Mundstück 15.

Die Steuerkulisse 34 weist vorzugsweise zwei - vorzugsweise mit der Spitze zueinander weisende - Sektorabschnitte 34A und 34B auf, insbesondere wobei die Sektorabschnitte 34A, 34B jeweils eine Kulisse für das Eingriffselement 29 bilden.

Vorzugsweise liegt der Sektorabschnitt 34A bzw. dessen Kulisse radial weiter innen als der Sektorabschnitt 34B bzw. dessen Kulisse und/oder ist die Kulissenbahn des Sektorabschnitts 34A länger als die Kulissenbahn des Sektorabschnitts 34B.

Die Steuerkulisse 34 bzw. der Sektorabschnitt 34B bildet vorzugsweise einen sich in Umfangsrichtung erstreckenden Ringnutabschnitt 34C, der vorzugsweise partiell durch das in Umfangsrichtung relativ zur Steuerkulisse 34 bewegliche Steuerelement 35 begrenzt und nach innen hin öffenbar ist.

In der Ausgangsposition befindet sich das Eingriffselement 29 vorzugsweise in einer Ecke des Sektorabschnitts 34B bzw. am Anfang des Ringnutabschnitts 34C bzw. einer davon gebildeten Kreisbahn, vorzugsweise wobei eine Bewegung des Eingriffselements 29 relativ zur Abdeckung 18 in Richtung der Spitze des Sektorabschnitts 34B bzw. in radialer Richtung aufgrund einer insbesondere in der Abdeckung 18 gebildeten, vorzugsweise rampenartigen, Haltenase 34D nicht möglich ist.

Beim radialen Herausziehen der Abdeckung 18, also bei dem ersten Teil der Öffnungsbewegung bzw. bei der Radialbewegung R, werden das Eingriffselement 29 und der Mitnehmer 25 von der Haltenase 34D bzw. Steuerkulisse 34 mitgenommen und radial in Richtung des Mundstücks 15 bzw. in Hauptausgaberichtung H bewegt. Hierbei wird das Eingriffselement 29 entlang des Führungsschlitzes 32 verschoben. Fig. 12 zeigt die sich dann ergebende, erste Öffnungsstellung.

Im Ergebnis bewirkt die Radialbewegung R der Abdeckung 18 vorzugsweise eine Radialbewegung R der Fördereinrichtung 20 bzw. des Mitnehmers 25 und damit die gewünschte Radialbewegung R bzw. Förderung der zugeordneten Kapselkammer 4 (also der gerade mit der Fördereinrichtung 20 bzw. dem Mitnehmer 25 in Eingriff stehenden Kapselkammer 4) in die Ausgabeposition A bzw. Aufnahmekammer 19.

Vorzugsweise hält bzw. sichert die Fördereinrichtung 20 bzw. der Mitnehmer 25 bzw. das Angriffselement 27 die jeweilige Kapselkammer 4 in der Ausgabeposition A, insbesondere solange bis die Inhalation erfolgt ist bzw. bis der Inhalator 1 oder dessen Abdeckung 18 wieder geschlossen wird. Die Rückstellbewegung erfolgt vorzugsweise durch die Rückstellfeder(n) 26, worauf später noch näher eingegangen wird.

Nach Erreichen der ersten Öffnungsstellung wird die Abdeckung 18 aufgeschwenkt, also die Schwenkbewegung S ausgeführt.

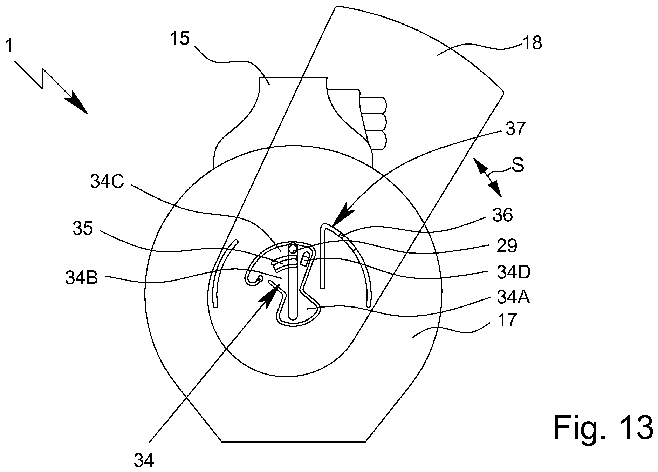

Fig. 13 zeigt eine Zwischenstellung zwischen der ersten Öffnungsstellung und der vollständig geöffneten zweiten Öffnungsstellung. Das Eingriffselement 29 wandert nun dem Ringnutabschnitt 34C bzw. der Kreisbahn entlang, vorzugsweise wobei die radial innen liegende Begrenzung sowie im weiteren Verlauf der Öffnungsbewegung bzw. der Kreisbahn das radial innen liegende Steuerelement 35 dafür sorgen, dass die Fördereinrichtung 20 bzw. der Mitnehmer 25 in der radial vorgeschobenen Stellung und damit auch die zugeordnete Kapselkammer 4 in der Ausgabeposition A gehalten werden bzw. eine Rückstellbewegung des Eingriffselement 29 aus dem Ringnutabschnitt 34C in den Sektorabschnitt 34A verhindert wird.

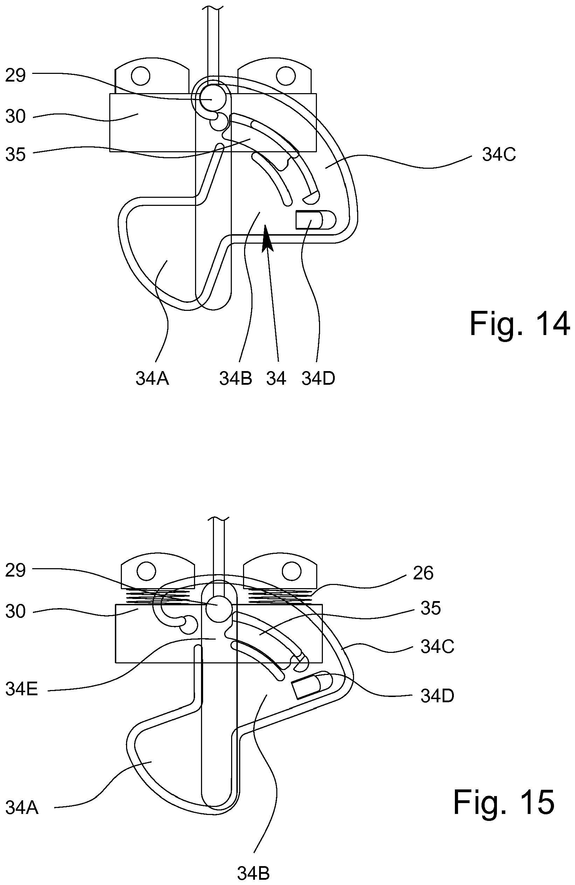

Fig. 14 zeigt den Zustand bei vollständig geöffneter Abdeckung 18, also bei Erreichen der zweiten Öffnungsstellung. Das Eingriffselement 29 befindet sich jetzt am Endpunkt der Kreisbahn bzw. des Ringnutabschnitts 34C und wird dort weiterhin gegen eine radiale Rückbewegung nach innen von der inneren Seitenwandung des Ringnutabschnitts 34C gehalten.

Es ist anzumerken, dass die innere Wandung des Ringnutabschnitts 34C partiell unterbrochen ist bzw. eine Unterbrechung 34E aufweist, vorzugsweise wobei die Unterbrechung 34E breiter als das Eingriffselement 29 bzw. dessen Durchmesser ist. Vorzugsweise beträgt der Mittelpunktswinkel der Unterbrechung 34E mehr als 5° oder 10° und/oder weniger als 20° oder 15°.

Beim Öffnungsvorgang wird diese Unterbrechung 34E durch das Steuerelement 35 überbrückt, so dass beim Öffnen der Abdeckung 18 durch Aufschwenken der Mitnehmer 25 kontinuierlich in seiner radial vorgeschobenen Position gehalten wird. Beim Schließvorgang bzw. Zurückschwenken der Abdeckung 18 wird das Steuerelement 35 vom Mitnehmer 25 bzw. Eingriffselement 29 in Richtung Haltenase 34D gedrückt, insbesondere derart, dass die Unterbrechung 34E freigegeben wird bzw. das Eingriffselement 29 vorzugsweise durch Federkraft bzw. selbsttätig nach unten bzw. radial nach innen bzw. in den Sektorabschnitt 34A bewegt wird, wie im Folgenden noch näher erläutert wird.

In der geöffneten Position der Abdeckung 18 ist die Öffnungseinrichtung 6 bzw. deren Betätigungselement 9 frei zugänglich. Nunmehr kann das Öffnen bzw. Anstechen der sich in der Ausgabeposition befindlichen Kapsel 3 durch Betätigung des Betätigungselements 9 erfolgen.

Nach dem Öffnen bzw. Anstechen der Kapsel 3 erfolgt das Inhalieren.

Anschließend wird der Inhalator 1 bzw. dessen Abdeckung 18 wieder geschlossen.

Fig. 15 zeigt einen Zwischenzustand kurz nach dem anfänglichen Schließen der Abdeckung 18. Das Eingriffselement 29 hat gerade die Unterbrechung 34E erreicht. Bei dieser Rückbewegung bzw. Schließbewegung wird das Steuerelement 35 zunächst von der Abdeckung 18 nicht mitgenommen, sondern kann sich relativ zu dieser bzw. dessen Schenkel 33 in einer Umfangsrichtung erstreckenden Führungsnut oder dergleichen verschieben, so dass die Unterbrechung 34E freigegeben wird und/oder das Eingriffselement 29 radial nach innen bewegt werden kann.

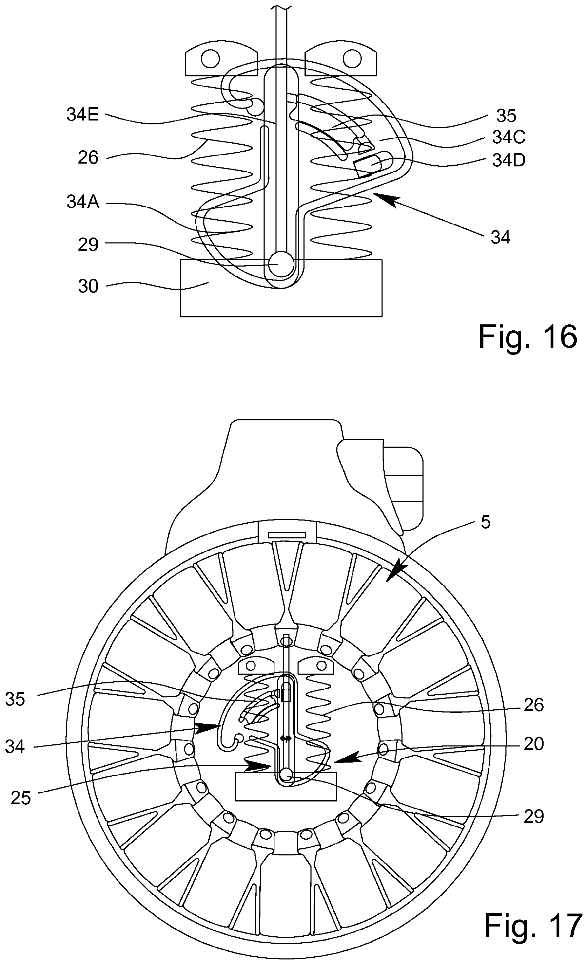

Durch die Kraft der Rückstellfeder(n) 26 wird der Mitnehmer 25 nun wieder in seine zurückgezogene Position zurückbewegt, also radial nach innen bewegt. Das Eingriffselement 29 wird also entlang des Führungsschlitzes 32 wieder radial nach innen bewegt. Hierbei bewegt sich das Eingriffselement 29 in den Sektorabschnitt 34B und anschließend in den sich anschließenden Sektorabschnitt 34A. Fig. 16 zeigt die Lage, die dann eingenommen wird.

Die Rückstellung des Mitnehmers 25 bewirkt auch die radiale Rückbewegung bzw. das Zurückbewegen der Kapselkammer 4 in das Magazin 5. Dies erfolgt vorzugsweise also durch Federkraft und/oder beim Schließen bzw. Zurückschwenken, insbesondere beim anfänglichen Schließen, der Abdeckung 18.

Fig. 17 zeigt den Zustand, wenn die Abdeckung 18 wieder in die erste Öffnungsstellung vollständig zurückgeschwenkt wurde. Das Eingriffselement 29 bewegt sich beim Zuschwenken entlang des äußeren Umfangs des Sektorabschnitts 34A bzw. dessen Kulisse in die in Fig. 17 gezeigte Position. Diese Position liegt jetzt radial gegenüber der in Fig. 11 gezeigten Anfangsposition des Eingriffselements 29 in dem anderen Sektorabschnitt 34B.

Beim radialen Einschieben der Abdeckung 18, also dem vollständigen Schließen der Abdeckung 18 (zum Einreichen der in Fig. 11 zu sehenden Stellung), verbleibt das Eingriffselement 29 in seiner zurückgezogenen radialen Lage im Führungsschlitz 32, bewegt sich jedoch relativ zu der Steuerkulisse 34 bzw. in den Sektorabschnitten 34A und 34B, wobei gegen Ende hin die Haltenase 34D überfahren wird, die insbesondere axial nach außen (ggf. zusammen mit dem Schenkel 33 bzw. der Abdeckung 18) ausfedert, bis die in Fig. 11 gezeigte Ausgangsposition wieder erreicht ist.

Die Haltenase 34D bzw. der davon gebildete Hinterschnitt oder Anschlag sorgt dann dafür, dass beim späteren radialen Herausbewegen der Abdeckung 18 das Eingriffselement 29 radial mitgenommen bzw. mitbewegt wird, also radial nach außen gezogen wird, um dementsprechend den Mitnehmer 25 und die zugeordnete (nächste) Kapsel 3 bzw. Kapselkammer 4 radial zur Ausgabeposition A bzw. in die Ausgabeposition A zu bewegen.

Vorzugsweise wird beim radialem Herausschieben der Abdeckung 18 folglich eine neue Kapsel 3 bzw. Kapselkammer 4 in die Ausgabeposition A gebracht bzw. für die Ausgabe bereitgestellt, insbesondere durch eine formschlüssige Verbindung zwischen der Abdeckung 18 bzw. deren Haltenase 34D und dem Mitnehmer 25 bzw. dessen Eingriffselement 29. Auf diese Weise beginnt ein neuer Betätigungszyklus.

Vorzugsweise wird der Mitnehmer 25 bzw. das Eingriffselement 29 auf beiden Seiten von der Abdeckung 18 bzw. deren Schenkeln 33 bzw. entsprechenden Steuerkulissen 34 jeweils wie voranstehend erläutert geführt und bewegt.

Im Folgenden wird das Weiterbewegen bzw. Weiterdrehen des Magazins 5 bzw. die Kopplung der Schwenkbewegung der Abdeckung 18 mit dem Magazin 5 näher erläutert.

Um von einer Kapsel 3 bzw. Kapselkammer 4 zur nächsten Kapsel 3 bzw. Kapselkammer 4 zu wechseln bzw. die nächste Kapsel 3 bzw. Kapselkammer 4 für die Abgabe bereitzustellen, ist das Magazin 5 vorzugsweise drehbar bzw. antreibbar, insbesondere durch Schwenken der Abdeckung 18.

Das Weiterbewegen bzw. Weiterdrehen des Magazins 5 zur nächsten Kapselkammer 4 erfolgt vorzugsweise beim Schließen der Abdeckung 18, insbesondere durch die Schließbewegung bzw. das Schwenken in Schließrichtung, besonders bevorzugt gegen Ende der Schwenkbewegung S beim Schließen, kurz vor Erreichen der ersten Öffnungsstellung und/oder unmittelbar nach Freigabe der Unterbrechung 34E durch Verschieben des Steuerelements 35 und/oder unmittelbar nach der Rückstellbewegung des Mitnehmers 25 bzw. nach der Bewegung der verwendeten Kapsel 3 bzw. Kapselkammer 4 zurück in das Magazin 5.

Insbesondere bewirkt die Schwenkbewegung S der Abdeckung 18 in Schließrichtung zunächst bzw. bei der anfänglichen Schwenkbewegung S eine Freigabe der Unterbrechung 34E bzw. ein Rückstellen des Mitnehmers 25 bzw. ein Zurückführen der verwendeten Kapsel 3 bzw. Kapselkammer 4 zurück in das Magazin 5 und erst anschließend bzw. bei entsprechendem Weiterschwenken bzw. gegen Ende der Schwenkbewegung S ein Weiterbewegen bzw. Weiterdrehen des Magazins 5 zur nächsten Kapsel 3 bzw. Kapselkammer 4. Auf diese Weise kann mittels einer - vorzugsweise ununterbrochenen und/oder gleichmäßigen - Schwenkbewegung S sowohl ein Zurückführen der verwendeten Kapsel 3 bzw. Kapselkammer 4 zurück in das Magazin 5 als auch ein Weiterbewegen des Magazins 5 erzielt werden, insbesondere ohne dass sich die beiden Bewegungsabläufe überlagern.

Die Abdeckung 18 wird beim Öffnen oder Schließen, vorzugsweise insgesamt um mehr als 60°, insbesondere im Wesentlichen 80 bis 90° oder mehr geschwenkt. Das Weiterbewegen bzw. Weiterdrehen des Magazins 5 erfolgt jeweils nur um einen wesentlich geringeren Winkel, beim Darstellungsbeispiel mit insgesamt 30 Kapselkammern 4 jeweils nur um 12°. Dementsprechend ist eine nur teilweise bzw. temporäre Kopplung der Schwenkbewegung S der Abdeckung 18 mit dem Magazin 5 erforderlich und gewünscht. Weiter kann dadurch die anfängliche Schwenkbewegung S der Abdeckung 18 beim Schließen für die Freigabe bzw. Bewirkung der (radialen) Rückbewegung der in der Ausgangsposition A befindlichen Kapselkammer 4 (mit entleerter Kapsel 3) in das Magazin 5 erfolgen.

Die Führungskulisse 37 ist vorzugsweise durch eine schlitzartige Durchbrechung im Gehäuse 17 gebildet. Sie weist insbesondere einen linearen Radialabschnitt 37A und einen sich daran in Umfangsrichtung anschließenden Bogenabschnitt 37B auf, wie in dem Ausschnitt gemäß Fig. 18 angedeutet.

Vorzugsweise greift die Abdeckung 18 bzw. deren Schenkel 33 ausgehend von den beiden Flachseiten des Inhalators 1 jeweils mit einem Antriebselement 36 (ausgebildet innen an Abdeckung 18) in jeweils eine zugeordnete Führungskulisse 37 ein und dient dem gewünschten Antrieb des Magazins 5, wobei die Antriebselemente 36 gegebenenfalls auch alternativ, abwechselnd an beiden Seiten am Magazin 5 angreifen können, um das Magazin 5 in gewünschter Weise, hier insbesondere in 12°-Schritten, antreiben zu können.

Das Antriebselement 36 ist vorzugsweise innen an der Abdeckung 18 bzw. auf einer dem Gehäuse 17 zugewandten Seite angeordnet.

Vorzugsweise ist das Antriebselement 36 einstückig mit der Abdeckung 18 ausgebildet und/oder an die Abdeckung 18 angeformt. Das Antriebselement 36 ist vorzugsweise zumindest teilweise flexibel ausgebildet und/oder schräg zu einem Schenkel 33 ausgerichtet, vorzugsweise derart, dass das Antriebselement 36 ausschließlich in eine Bewegungsrichtung form- und/oder kraftschlüssig mit dem Antriebsabschnitt 38 verbindbar ist und/oder eine ratschenartige Verbindung zwischen dem Antriebselement 36 und dem Antriebsabschnitt 38 gebildet wird.

Die Führung des Antriebselements 36 der Abdeckung 18 in der Führungskulisse 37 und/oder der Eingriff des Eingriffselements 29 in die Steuerkulisse 34 kann jeweils einer gewünschten bzw. erforderlichen Lagerung der Abdeckung 18 am Inhalator 1 bzw. Gehäuse 17 dienen. Jedoch sind alternativ oder zusätzlich auch sonstige konstruktive Lösungen für die Lagerung einsetzbar.

Fig. 11 bis 13 veranschaulichen die Bewegungen eines Antriebselements 36 in der zugeordneten Führungskulisse 37 während dem des Öffnens der Abdeckung 18 bzw. bei den verschiedenen Zuständen.

Fig. 18 veranschaulicht den Angriff des Antriebselements 36 am Magazin 5, insbesondere an einem Antriebsabschnitt 38 des Magazins 5, um dieses weiterzudrehen. Bei dem Antriebsabschnitt 38 handelt es sich insbesondere um einen axial vor- oder zurückspringenden Anschlag, so dass das in Umfangsrichtung auflaufende Antriebselement 36 formschlüssig in Eingriff treten kann, um das Magazin 5 bei der Schließbewegung - also während der Bewegung entlang eines Teils des Bogenabschnitts 37B - drehen zu können.

Vorzugsweise ist der Antriebsabschnitt 38 teilkreisförmig ausgebildet. Hier sind jedoch auch andere Lösungen möglich.

Durch die anschließende Radialbewegung R beim radialen Einwärtsbewegen entlang des Radialabschnitts 37A - also beim radialen Schließen der Abdeckung 18 - wird das Antriebselement 36 von dem Antriebsabschnitt 38 bzw. den davon gebildeten Anschlag gelöst oder außer Eingriff gebracht.

Beim Öffnen der Abdeckung 18 wird das Antriebselement 36 in entgegengesetzter Richtung bewegt. Es wird über den nächsten Antriebsabschnitt 38 - vorzugsweise mittels einer axial geneigten schiefen Ebene oder Gleitfläche, die insbesondere auch durch eine Kapselkammer 4 gebildet sein kann - hinweg bewegt, vorzugsweise derart, dass das Antriebselement 36 bzw. die Abdeckung 18 und deren Schenkel 33 und/oder der jeweilige Antriebsabschnitt 38 axial ausweichen bzw. ausfedern können bzw. kann, um das Überfahren zu ermöglichen.

Falls die Öffnungsbewegung der Abdeckung 18 derart groß ist, dass ein weiterer Antriebsabschnitt 38 am Magazin 5 von dem Antriebselement 36 überfahren wird, wird das Antriebselement 36 vorzugsweise von der Führungskulisse 37 axial derart angehoben bzw. ausgerückt, dass ein vorzeitiger Eingriff bei der Schließbewegung mit dem weiteren Antriebsabschnitt 38 nicht erfolgt.

Beim Schließen der Abdeckung 18, also beim Zuschwenken, erfolgt dann der gewünschte Eingriff an dem einen überfahrenen Antriebsabschnitt 38 mit dem Antriebselement 36, um das Magazin 5 um eine weitere Position zu takten, also zur nächsten Kapsel 3 und Kapselkammer 4 (jetzt in der jeweils anderen Ebene E1 bzw. E2) weiterzudrehen.

Vorzugsweise weist der Inhalator 1 eine Einrichtung zur Sicherung des Magazins 5 gegen unerwünschtes Zurückdrehen und/oder zum jeweils definierten Positionieren des Magazins 5 in den verschiedenen Drehlagen (zur einzelweisen Ausrichtung der Kapselkammern 4 zur Aufnahmekammer 19 bzw. zum Mundstück 15 bzw. zur Ausgabeposition A) auf. Beim Darstellungsbeispiel sind hierzu vorzugsweise axial und in Umfangsrichtung Aussparungen 39 mit vorlaufenden Rippen 40 und nachlaufenden Rippen 41 am Magazin 5 bzw. dessen Träger 21 gebildet, wie in Fig. 19 angedeutet, die mit vorzugsweise knopfartigen und/oder axialen Vorsprüngen 42 am Gehäuse 17 derart zusammenwirken, dass in die gewünschten Drehpositionen jeweils eine oder mehrere Vorsprünge 42 in die Aussparungen 39 eingreifen und die Rippen 40, 41 ein Weiterdrehen und/oder Zurückdrehen durch entsprechende Anlage am zugeordneten Vorsprung 42 verhindern und/oder durch asymmetrische Formgebung und/oder Verformbarkeit der Rippen 40, 41 ein Weiterdrehen in nur eine Richtung ermöglicht wird. Jedoch sind auch andere konstruktive Lösungen möglich.

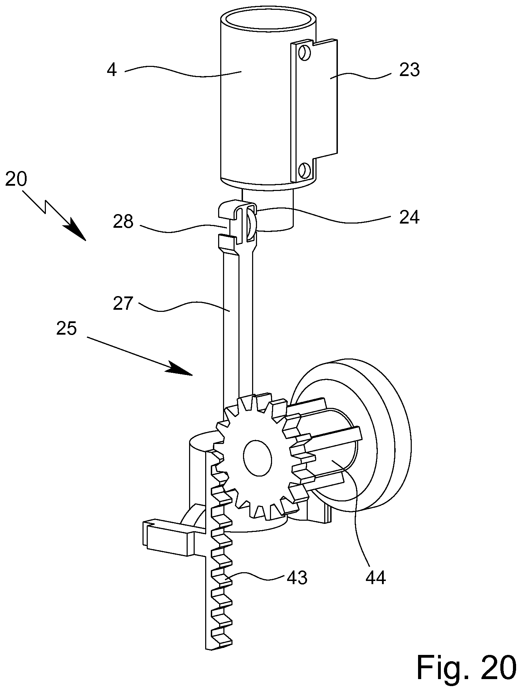

Fig. 20 zeigt in der perspektivischen Darstellung schematisch eine alternative Ausbildung der Fördereinrichtung 20 bzw. des Mitnehmers 25. Hier ist der Mitnehmer 25 zahnstangenartig ausgebildet bzw. mit einer Zahnstange 43 versehen, die über ein von der Abdeckung 18 in geeigneter Weise antreibbares Zahnrad 44 in gewünschter Weise linear bzw. in radialer Richtung bewegbar ist.

Vorzugsweise ist bei einer derartigen Ausführungsform das Zahnrad 44 mit der Abdeckung 18 bzw. dessen Schenkel 33 form- und/oder kraftschlüssig verbunden bzw. gekoppelt, insbesondere derart, dass eine Schwenkbewegung der Abdeckung 18 zu einer Rotation des Zahnrads 44 bzw. radialen Bewegung des Mitnehmers 25 führt.

Insbesondere ist bei einer derartigen Ausführungsform ausschließlich eine Schwenkbewegung der Abdeckung 18 vorgesehen bzw. wird die Schwenkbewegung der Abdeckung 18 in eine radiale und/oder lineare Bewegung des Mitnehmers 25 umgesetzt, vorzugsweise derart, dass eine radiale Bewegung der Abdeckung 18 und/oder des Zahnrads 44 entfällt.

Vorzugsweise wird das Zahnrad 44 nur abschnittsweise bei einer Schwenkbewegung der Abdeckung 18 von der Abdeckung 18 angetrieben bzw. ist das Zahnrad 44 nur abschnittsweise bzw. in einem definiertem Winkelbereich mit der Abdeckung 18 form- und/oder kraftschlüssig verbunden, beispielsweise über eine Kupplung mit Freilauf und/oder eine selbstlösende Kupplung. So kann die Verbindung zwischen der Abdeckung 18 und dem Zahnrad 44 beispielsweise durch eine Schwenkbewegung S zum Schließen des Inhalators 1 gelöst werden und der Mitnehmer 25 mittels Federkraft radial nach innen bewegt werden. Auf diese Weise kann - analog zu der ersten Ausführungsform - mittels einer - vorzugsweise ununterbrochenen und/oder gleichmäßigen - Schwenkbewegung S sowohl ein Zurückführen der verwendeten Kapsel 3 bzw. Kapselkammer 4 zurück in das Magazin 5 als auch ein Weiterbewegen des Magazins 5 erzielt werden, insbesondere ohne dass sich die beiden Bewegungsabläufe überlagern.

Nachfolgend werden weitere Ausführungsformen des vorschlagsgemäßen Inhalators 1 und des vorschlagsgemäßen Magazins 5 erläutert, wobei die bisherigen Ausführungen und Erläuterungen insbesondere bezüglich der ersten Ausführungsform auch für die weiteren Ausführungsformen entsprechend oder ergänzend gelten, auch wenn entsprechende Wiederholungen weggelassen sind. Die nachfolgenden Ausführungen und Erläuterungen beschränken sich daher insbesondere auf wesentliche neue Aspekte bzw. auf Unterschiede.

Fig. 21 zeigt eine zweite Ausführungsform des vorschlagsgemäßen Inhalators 1 in einem Schnitt senkrecht zur Hauptebene E, also senkrecht zu dem Schnitt gemäß Fig. 5.

Bei der zweiten Ausführungsform ist der Mitnehmer 25 anders ausgebildet, insbesondere als ein Stanz- bzw. Biegeteil aus Metall.

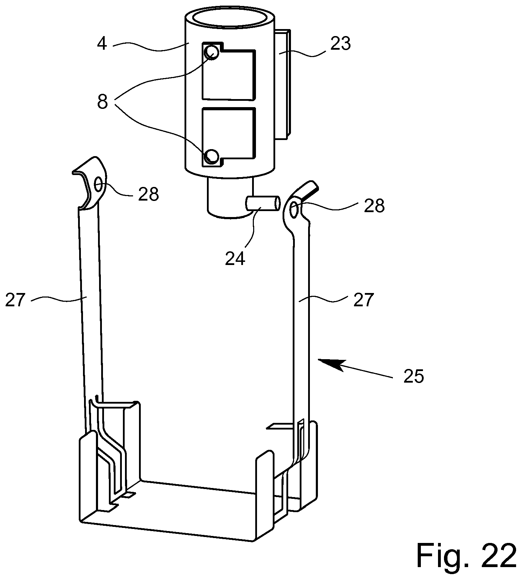

Fig. 22 zeigt den Mitnehmer 25 im nicht eingebauten Zustand mit einer zugeordneten, jedoch noch nicht in Eingriff stehenden Kapselkammer 4.

Der Mitnehmer 25 ist wiederum über ein Eingriffselement 29, das mit dem Mitnehmer 25 gekoppelt und nur in Fig. 21 gezeigt ist, in radialer Richtung im Inhalator 1 bzw. Gehäuse 17 verschiebbar, um die Kapselkammern 4 einzelweise aus dem Magazin 5 (zumindest im Wesentlichen) radial in die Aufnahmekammer 19 bzw. Ausgabeposition A hin- und vorzugsweise zurückzubewegen.

Der Mitnehmer 25 weist hier zwei axial beabstandete und in axialer Richtung nach innen bzw. zueinander biegbare Angriffselemente 27 mit jeweils einer Ausnehmung 28 im Bereich des freien Endes auf.

Fig. 21 und 22 zeigen den Ausgangszustand. Hier befindet sich die Fördereinrichtung 20 bzw. der Mitnehmer 25 noch in der radial zurückgezogenen Position. Das jeweilige Angriffselement 27 und die jeweilige Kapselkammer 4 stehen noch nicht in Eingriff miteinander.

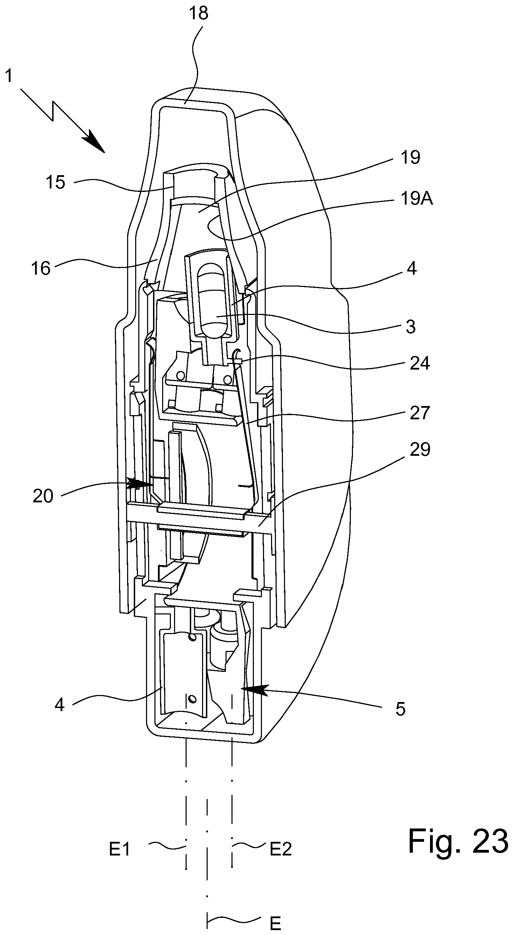

Fig. 23 zeigt in einem zu Fig. 21 korrespondierenden Schritt einen Zwischenzustand bei teilweiser Radialbewegung R der Abdeckung 18 und des Mitnehmers 25. Das entsprechende Angriffselement 27, hier das rechte Angriffselement 27, wurde durch das Gehäuse 17 oder ein sonstiges Bauteil axial nach innen abgelenkt und hierdurch mit der zugeordneten Kapsel 4 in Eingriff gebracht, so dass der Angriffsabschnitt 24 dieser Kapselkammer 4 in die Ausnehmung 28 dieses Angriffselements 27 eingreift.

Weiter ist in Fig. 23 zu erkennen, dass die Kapselkammer 4 bereits teilweise aus dem Magazin 5 und ihrer Ebene E2 herausbewegt worden ist und hierbei leicht zur Hauptebene E gekippt wird. Hierbei dient insbesondere das Mundstück 15 bzw. der Verbindungsabschnitt 16 bzw. die innere Wandung der Aufnahmekammer 19 als Führung, um die jeweilige Kapselkammer 4 aus der jeweiligen Ebene E1 bzw. E2 in die Hauptebene E zu führen und schließlich in gewünschter Weise - insbesondere in der Ebene E bzw. in Verlängerung des Mundstücks 15 oder der Hauptausgaberichtung H - auszurichten.

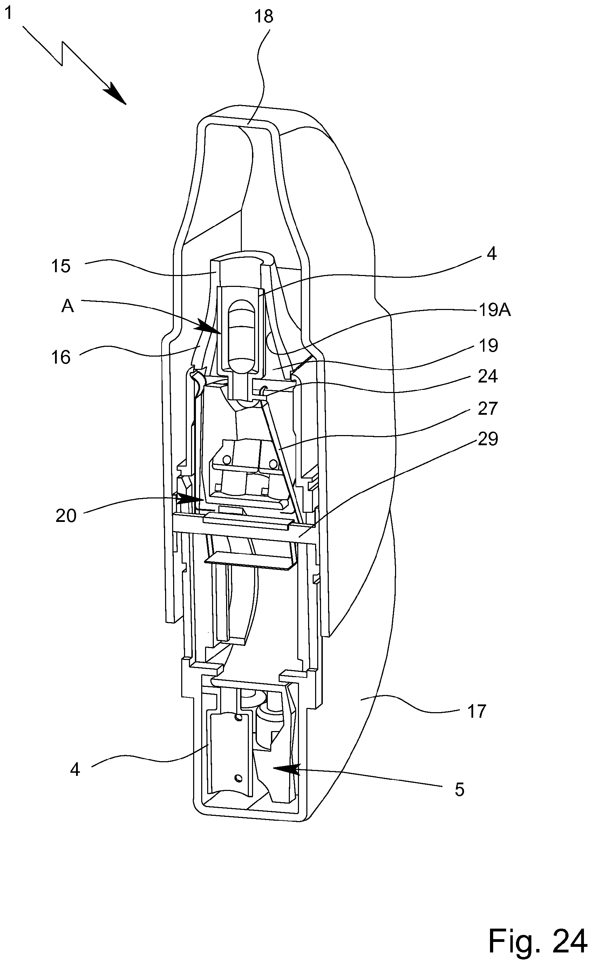

Fig. 24 zeigt dann den entsprechenden Endzustand bei Erreichen der ersten Öffnungsstellung, also bei radial vollständig ausgezogener Abdeckung 18 und bei in die Ausgabeposition A bewegter Kapselkammer 4. Das jeweilige Antriebselement 27 hat sich hierbei weiter axial zur Hauptebene E hin bewegt, insbesondere durch entsprechendes Führen der Kapselkammer 4 in der Aufnahmekammer 19 und/oder durch die Führung 23 und/oder auf sonstige Art und Weise.

Nunmehr kann das weitere Öffnen durch Aufschwenken der Abdeckung 18 erfolgen.

Nach dem Inhalieren und Anstechen wird die Abdeckung 18 wieder geschlossen. Bei der radialen Rückbewegung wird der Mitnehmer 25 wieder in seine Ausgangsstellung, also in seine radial zurückgezogene Stellung zurückbewegt. Hierbei wird die in Eingriff mit dem zugeordneten Antriebselement 27 stehende Kapselkammer 4 wieder aus der Ausgangsposition A in das Magazin 5 zurückbewegt bzw. zurückgezogen.

Bei der zweiten Ausführungsform erfolgt das Weiterdrehen des Magazins 5 vorzugsweise kurz vor Erreichen der Ausgangsposition, also beim radialen Zurückbewegen der Abdeckung 18, wobei der Mitnehmer 25 bzw. dessen Angriffselement 27 bereits kurz vorher von der in das Magazin 5 zurückbewegten Kapselkammer 4 - insbesondere auch durch entsprechendes axiales Ausfedern des Angriffselements 27 - entkoppelt wird. Das Weiterdrehen des Magazins 5 erfolgt dann durch Federkraft, wobei lediglich die Freigabe bei Erreichen der Ausgangsstellung zum Weiterdrehen um eine Kapselkammer 4 ausgelöst wird. Die nicht näher dargestellte Federmechanik kann beispielsweise durch eine Uhren- oder Schenkelfeder realisiert werden, die ausreichend Energie besitzt, um das Magazin 5 in gewünschter Weise zu drehen. Alternativ kann auch eine Federmechanik eingesetzt werden, die jeweils beim Öffnen und/oder beim Schließen der Abdeckung 18, insbesondere beim Schwenken der Abdeckung 18, neu gespannt wird und sich beim Weiterdrehen des Magazins 5 jeweils wieder entsprechend entspannt.

Bei der zweiten Ausführungsform des Inhalators 1 ist das Magazin 5 vorzugsweise zumindest im Wesentlichen entsprechend der ersten Ausführungsform ausgebildet, jedoch an die andersartige Ausbildung des Mitnehmers 25 angepasst, insbesondere um den außenseitigen Angriff jeweils eines Angriffselements 27 an einer zugeordneten Kapselkammer 4 zu ermöglichen und das andere Angriffselement 27 auf der anderen Seite jeweils außen vorbeizuführen. Weiter müssen die Kapselkammern 4 mit ihren Angriffsabschnitten 24 nach außen weisen, um den gewünschten Formschluss mit dem zugeordneten Angriffselement 27 durch Eingriff in dessen Ausnehmung 28 zu ermöglichen. Diese Modifikation stellt die zweite Ausführungsform des vorschlagsgemäßen Magazins 5 dar.

Nachfolgend wird anhand der weiteren Figuren eine dritte Ausführungsform des vorschlagsgemäßen Inhalators 1 sowie eine dritte Ausführungsform des vorschlagsgemäßen Magazins 5 näher erläutert.

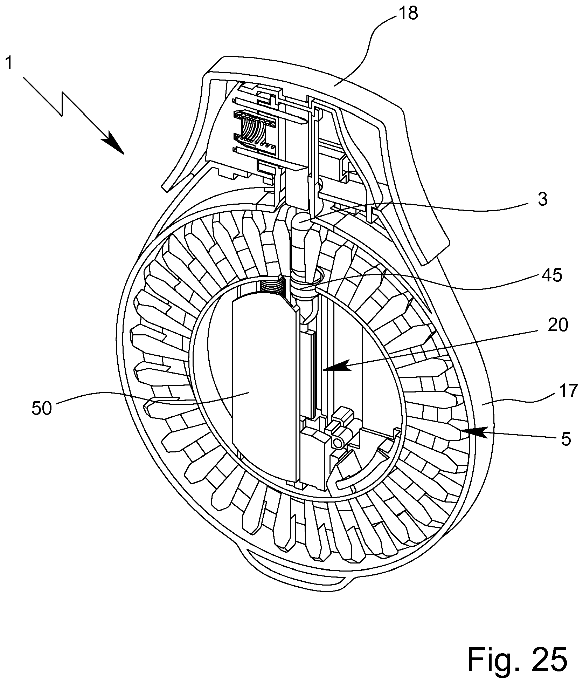

Fig. 25 zeigt den Inhalator 1 in einem schematischen Schnitt entsprechend Fig. 5 im Ausgangszustand, also mit geschlossener Abdeckung 18. Bei der dritten Ausführungsform sind lediglich die Kapseln 3, das heißt ohne Kapselkammern 4, aus dem Magazin 5 zumindest im Wesentlichen radial in die Ausgabeposition A bzw. in eine Aufnahmekammer 19 bzw. eine Kapselkammer 4 bewegbar, um dort geöffnet bzw. angestochen zu werden. Die gemeinsame Kapselkammer 4 muss dementsprechend nach dem Hineinbewegen der jeweiligen Kapsel 3 geschlossen und zur Entnahme der entleerten Kapsel 3 wieder geöffnet werden. Dies erfolgt vorzugsweise mit Hilfe des (jeweiligen) Mitnehmers 25.

In Fig. 25 ist aus Veranschaulichungsgründen lediglich eine Kapsel 3 in dem Magazin 5 angedeutet, der Träger 21 also ansonsten nicht gefüllt.

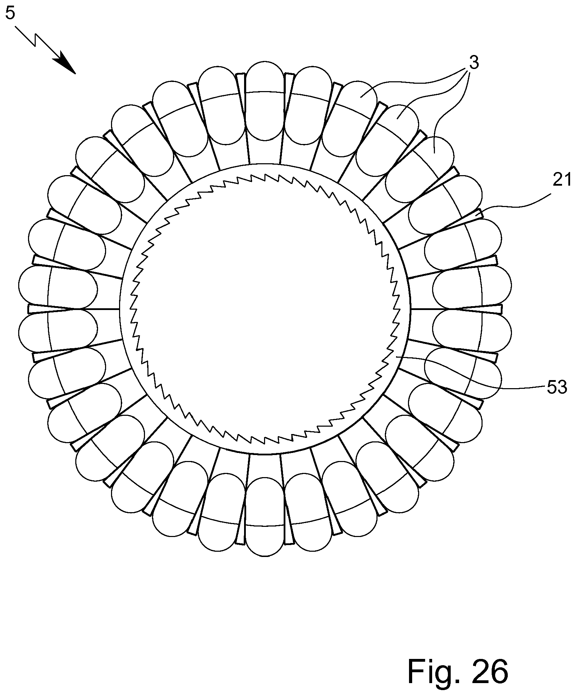

Das Magazin 5 gemäß der dritten Ausführungsform ist schematisch in Fig. 26 dargestellt. Da das Magazin 5 keine Kapselkammern 4 enthält, kann es kompakter ausgebildet sein bzw. mehr Kapseln 3 aufnehmen, als dies bei der Aufnahme von Kapseln 3 mit zugeordneten Kapselkammern 4 der Fall ist. Beim Darstellungsbeispiel weist das Magazin 5 vorzugsweise 30 Kapseln 3 in einer Ringanordnung bzw. Ebene auf. Vorzugsweise weist das Magazin 5 zwei Ringanordnungen von Kapseln 3 wiederum in axial versetzten Ebenen E1 und E2 auf. Insgesamt weist das Magazin 5 vorzugsweise in jeder Ringanordnung bzw. Ebene E1, E2, 30 Kapseln 3, insbesondere also 60 Kapseln 3 auf.

Die Ringanordnungen der Kapseln 3 sind vorzugsweise wiederum in Umfangsrichtung versetzt zueinander angeordnet, wie bei den ersten beiden Ausführungsformen des Magazins 5. Aufgrund der höheren Anzahl der Kapseln 3 ergeben sich jedoch kleinere Taktschritte bzw. Taktwinkel beim Weiterdrehen bzw. Vorwärtsbewegen des Magazins 5 zur nächsten Kapsel 3. Insbesondere beträgt der Drehwinkel bei jeder Taktung bzw. jedem Weiterdrehen beim Darstellungsbeispiel nur 6° gegenüber den bei der ersten und zweiten Ausführungsform erforderlichen 12°. Jedoch hängen diese Winkel von der jeweils tatsächlich vorgesehenen Anzahl von Kapseln 3 sowie deren Verteilung über den Umfang des Magazins 5 ab.

Vorzugsweise sind die Kapseln 3 klemmend im Magazin 5 bzw. dessen Träger 21 gehalten.

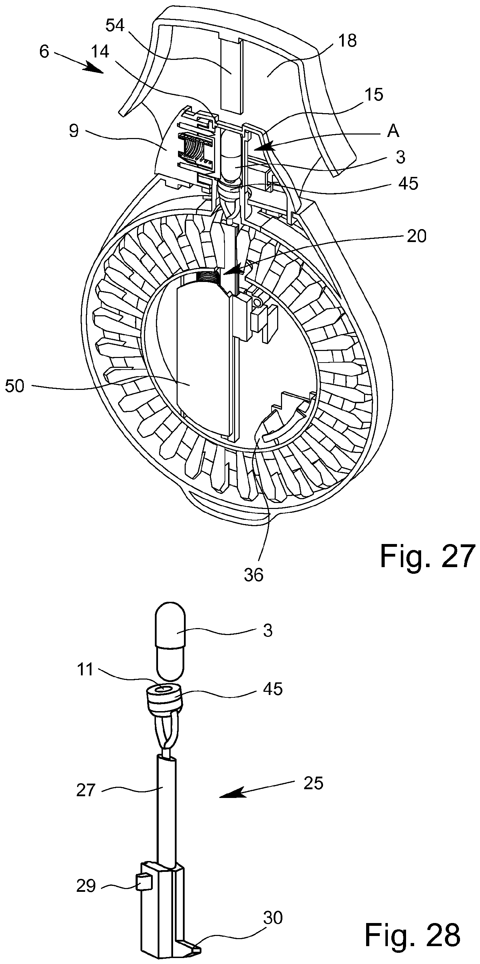

Fig. 27 zeigt den Inhalator 1 in einem schematischen Schnitt ähnlich Fig. 25 jedoch im ersten Öffnungszustand, also bei radial herausgezogener Abdeckung 18. Die entsprechende Kapsel 3 befindet sich in der Ausgabeposition A bzw. in der Kapselkammer 4. Der zugeordnete Mitnehmer 25 hat die hier im Mundstück 15 bzw. dessen Verbindungsabschnitt 16 gebildeten Kapselkammer 4 einlassseitig verschlossen, vorzugsweise wobei der Mitnehmer 25 jedoch einen Einlass 11 aufweist oder bildet, so dass Luft in die Kapselkammer 4 einströmen kann.

Fig. 28 zeigt in einer schematischen Darstellung eine bevorzugte Ausgestaltung des Mitnehmers 25 mit zugeordneter Kapsel 3. Insbesondere weist der Mitnehmer 25 an seinem Angriffselement 27 einen angepassten, vorzugsweise ringförmigen, Kopf 45 auf, der Kapselkammer 4 verschließt und/oder dessen Einlass 11 bildet, vorzugsweise bodenseitig.

Vorzugsweise weist der Mitnehmer 25, insbesondere der Kopf 45, eine Durchbrechung bzw. Bohrung als Einlass 11 auf. Hier sind jedoch auch andere Lösungen möglich.

Der Kopf 45 ist vorzugsweise beweglich oder geneigt bzw. neigbar.

Das Angriffselement 27 des Mitnehmers 25 ist vorzugsweise axial (bezüglich des Inhalators 1) bzw. quer auslenkbar, um die in der Hauptebene E befindliche Kapsel 3 mit dem Kopf 45 definiert und insbesondere ohne Verkanten in die Ausgabeposition A schieben zu können, insbesondere so dass der im Kopf 45 gebildete Einlass 11 geradlinig zu der Längserstreckung der Kapselkammer 4 bzw. der Hauptausgaberichtung H ausgerichtet ist.

Der Inhalator 1 bzw. dessen Fördereinrichtung 20 weist bei der dritten Ausführungsform vorzugsweise zwei unabhängig voneinander bzw. abwechselnd bewegbare Mitnehmer 25 zur abwechselnden Bewegung jeweils einer Kapsel 3 aus einer der Ringanordnungen bzw. Ebenen E1 oder E2 in die Ausgabeposition A bzw. in die gemeinsame Kapselkammer 4 und insbesondere wieder zurück in das Magazin 5 auf.

Zum abwechselnden Antrieb der Mitnehmer 25 weist die Fördereinrichtung 20 vorzugsweise einen Stellschieber 46 auf, der in Abhängigkeit von seiner Axiallage wahlweise mit einem der Mitnehmer 25 in Eingriff bringbar ist.

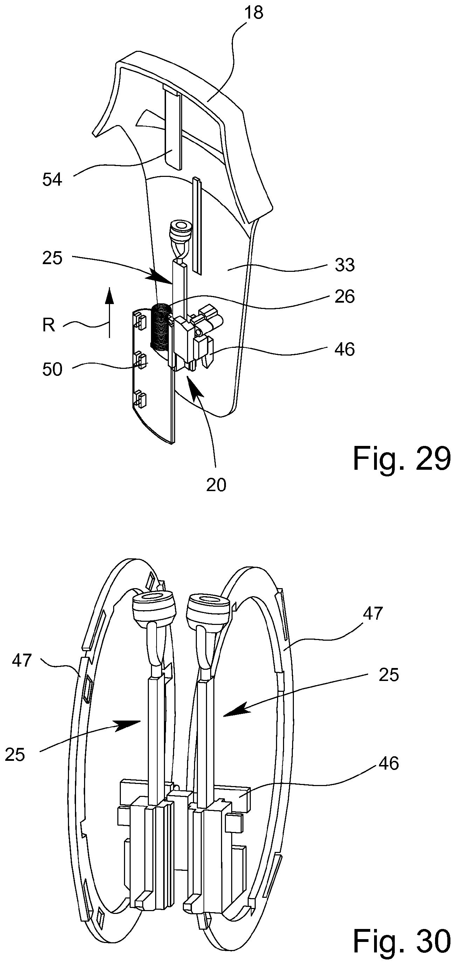

Der Stellschieber 46 ist vorzugsweise axial verschieblich mit der Abdeckung 18 bzw. deren Schenkel 33 gekoppelt, wie schematisch in Fig. 29 für eine Seite der Abdeckung 18 und eine Hälfte des Stellschiebers 46 angedeutet. Der Stellschieber 46 erstreckt sich also zwischen den beiden Schenkeln 33 und ist zwischen diesen axial und/oder radial verschieblich gehalten bzw. geführt. Weiter ist der Stellschieber 46 vorzugsweise drehfest im Hinblick auf die Schwenkbewegung S mit der Abdeckung 18 bzw. deren Schenkeln 33 gekoppelt.

Der Inhalator 1 bzw. dessen Fördereinrichtung 20 weist eine Umschalteinrichtung zum axialen Verschieben bzw. Einstellen der Axiallage des Stellschiebers 46 auf. Beim Darstellungsbeispiel weist die Umschalteinrichtung zwei Steuerringe 47 auf, die zusammen mit dem Stellschieber 46 und dem zugeordneten Mitnehmern 25 schematisch in Fig. 30 angedeutet sind.

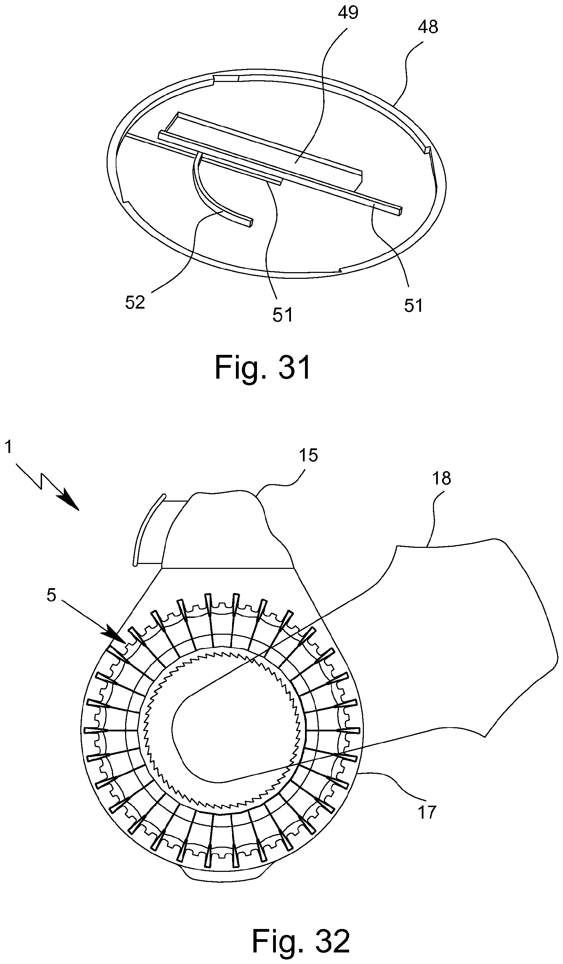

Der Inhalator 1 bzw. dessen Gehäuse 17 weist vorzugsweise auf beiden Flachseiten jeweils einen Deckel 48 auf, der in Fig. 31 perspektivisch dargestellt ist. Der Deckel 48 weist einen Bewegungsschlitz 49 auf, der die gewünschte Kopplung oder Verbindung des Stellschiebers 46 mit dem zugeordneten Schenkel 33 der Abdeckung 18 ermöglicht.

Die Deckel 48 sind drehbar am Gehäuse 17 angeordnet und drehfest mit der Abdeckung 18 bzw. deren Schenkel 33 gekoppelt, wobei jedoch die radiale Beweglichkeit bzw. Verschiebbarkeit der Abdeckung 18 erhalten bleibt.

Die Steuerringe 47 sind jeweils innen an den Deckeln 48 angeordnet und werden von diesen in einer Richtung drehend angetrieben. In der anderen Richtung sind die Steuerringe 47 gesperrt, weisen also relativ zu den Deckeln 48 einen Freilauf auf. Die Sperrung kann insbesondere durch entsprechenden Eingriff am Gehäuse 17 realisiert werden.

Die Steuerringe 47 werden immer in Schritten vorzugsweise von 90° weiter gedreht, insbesondere wenn die Abdeckung 18 beim Öffnen oder Schließen jeweils um 90° geschwenkt wird, wie dies bei den Darstellungsbeispielen bevorzugt der Fall ist.

Die Steuerringe 47 sind vorzugsweise 90° versetzt eingebaut und weisen jeweils an den entsprechenden Stellen Steuermittel auf, um den Stellschieber 46 in gewünschter Weise abwechselnd in die eine und in die andere Richtung axial zu verschieben bzw. zu verstellen. Hierauf wird später noch näher eingegangen.

Die beiden Mitnehmer 25 sind insbesondere mittels eines Führungselements 50 innerhalb des Magazins 5 in radialer Richtung R bzw. Hauptausgaberichtung H radial verschieblich geführt, wie in Fig. 29 für einen Mitnehmer 25 schematisch angedeutet.

Jeder Mitnehmer 25 weist vorzugsweise eine Federaufnahme 30 für eine zugeordnete Rückstellfeder 26 auf, die mit ihrem anderen Ende insbesondere über einen Lagerabschnitt 31 am Führungselement 50 abgestützt ist, um den jeweiligen Mitnehmer 25 in die untere bzw. radial eingeschobene Stellung, wie in Fig. 25 angedeutet, vorzuspannen bzw. zurückzubewegen.

Weiter weist jeder Mitnehmer 25 vorzugsweise ein Eingriffselement 29 auf, das hier vorzugsweise als seitlich vorstehende Nase ausgebildet ist, wie in Fig. 28 angedeutet. Mit diesem Eingriffselement 29 greift der Mitnehmer 25 in eine entsprechende Radialnut 51 im zugeordneten Deckel 48 ein, an die sich eine Bogennut 52 anschließt (vgl. Fig. 31), auf deren Funktion später noch näher eingegangen wird.

Ausgehend von der Ausgangsstellung des Inhalators 1 erfolgt beim Öffnen des Inhalators 1, also beim radialen Herausziehen der Abdeckung 18 eine entsprechende radiale Bewegung des Stellschiebers 46. In Abhängigkeit von der axialen Stellung nimmt der Stellschieber 46 einen der beiden Mitnehmer 25 mit und schiebt diesen gegen die Kraft der zugeordneten Rückstellfeder 26 mit in radialer Richtung zum Mundstück 15 hin, so dass dieser Mitnehmer 25 die sich in Ausrichtung mit dem Mundstück 15 befindende Kapsel 3 aus dem Magazin 5 zumindest im Wesentlichen radial herausschiebt und in die dem Mundstück 15 zugeordnete Kapselkammer 4 schiebt und diese mittels des Kopfes 45 verschließt, also einen Einlass 11 für die Kapselkammer 4, wie beispielsweise bei der Darstellung gemäß Fig. 1, bildet. Dieser Zustand ist in Fig. 27 dargestellt und entspricht der ersten Öffnungsstellung.

Anschließend wird die Abdeckung 18 zur Seite geschwenkt, hierbei wird der Stellschieber 46 innen am Magazin 5 bzw. in Umfangsrichtung mit bewegt. Somit kann der Stellschieber 46 den in radialer Richtung ausgefahrenen Mitnehmer 25 nicht mehr in dieser radialen Stellung halten. Doch wird beim Aufschwenken der Abdeckung 18 der entsprechende Deckel 48 mitgedreht, so dass das Eingriffselement 29 in die bereits genannte Bogennut 52 des Deckels 48 eingreift, wodurch der Mitnehmer 25 bei aufgeschwenkter Abdeckung 18 in der radial vorgeschobenen Stellung gehalten bzw. gesichert wird. Figur 32 zeigt einen solchen Zustand mit aufgeschwenkter Abdeckung 18.

Zum Antrieb bzw. Drehen des Magazins 5 weist der Inhalator 1 eine entsprechende Antriebseinrichtung auf. Diese umfasst, wie bei der ersten Ausführungsform, ein Antriebselement 36. Bei der ersten Ausführungsform ist ein Antriebselement 36 mit der Abdeckung 18 gekoppelt oder verbunden. Bei der dritten Ausführungsform ist das Antriebselement 36 hingegen unabhängig von der Abdeckung 18.

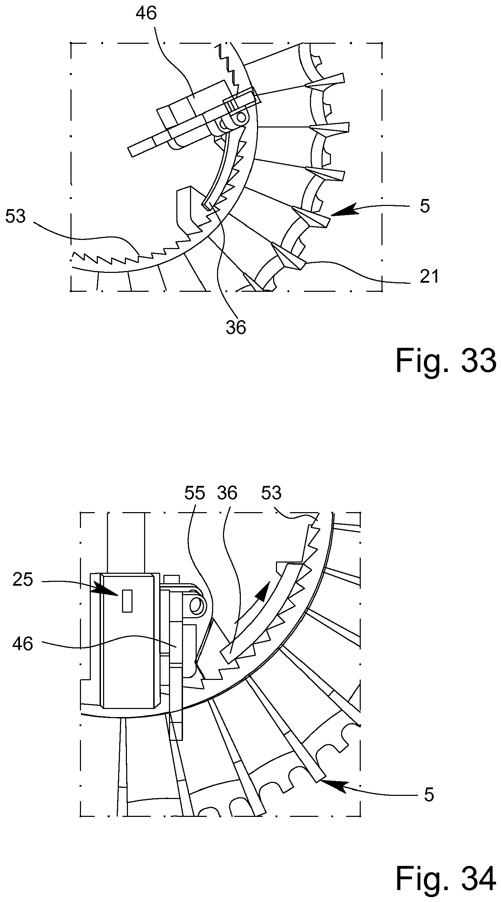

Bei der dritten Ausführungsform ist das Antriebselement 36 vorzugsweise innen am Magazin 5 angeordnet und mit einer Außenverzahnung versehen, die mit einer Innenverzahnung 53 am Magazin 5 bzw. dessen Träger 21 zusammenwirkt, wie in Fig. 33 angedeutet. Insbesondere erstreckt sich das Antriebselement 36 über einen gewissen Umfangsbereich der Innenverzahnung 53.

Weiter ist das Antriebselement 36 vorzugsweise radial nach außen vorgespannt, insbesondere durch Federkraft.

Die Innenverzahnung 53 und die entsprechende Außenverzahnung am Antriebselement 36 sind vorzugsweise sägezahnförmig und/oder derart ausgebildet, dass bei vollständigen Aufschwenken der Abdeckung 18 der mit bewegte Stellschieber 46 in Umfangsrichtung am Antriebselement 36 in Anlage kommt bzw. auf dieses einwirkt, um das Antriebselement 36 um einen Takt bzw. eine Rastung oder einen Zahn in Umfangsrichtung relativ zur Innenverzahnung 53 weiterzubewegen, um das spätere Weiterdrehen des Magazins 5 um eine Taktung um einen entsprechenden Winkelschritt, hier von vorzugsweise 6°, vorzubereiten.

Anschließend kann die Kapsel 3 durch Betätigen der Öffnungseinrichtung 6 bzw. des Betätigungselements 9 geöffnet werden und die Inhalation erfolgen.

Nach der Inhalation wird die Abdeckung 18 wieder zurück über das Mundstück 15 geschwenkt, also zurück in die erste Öffnungsstellung. Hier bieten sich nun zwei Alternativen an.

Entweder wird bei Erreichen der ersten Öffnungsstellung der in der ausgefahrenen Radialstellung befindliche Mitnehmer 25 bzw. dessen Eingriffselement 29 direkt freigegeben und aufgrund der Kraft der zugeordneten Rückstellfeder 26 unmittelbar wieder in die eingefahrene Stellung radial zurück bzw. nach innen bewegt.

Alternativ kommt aufgrund des Zurückschwenkens der Stellschieber 46 auch wieder in Kontakt mit dem in der ausgefahrenen Radialstellung sich befindenden Mitnehmer 25 und wird daher zusammen mit dem Mitnehmer 25 von dessen Rückstellfeder 26 wieder zurück in die Ausgangsposition bewegt. Bei dieser Rückbewegung wird dann entsprechend die Abdeckung 18 radial in die Ausgangsposition zurückgezogen.

Bei der radialen Rückbewegung der Abdeckung 18 in die Ausgangsposition, also beim vollständigen Schließen des Mundstücks 15, wird ein innen an der Abdeckung 18 angebrachter Dorn 54 (vgl. Fig. 29) radial in das Mundstück 15 von außen eingeführt und sorgt dafür, dass die sich in der Kapselkammer 4 befindliche, entleerte Kapsel 3 wieder in das zugeordnete Magazin 5, genauer gesagt in den Aufnahmebereich im Magazin 5, aus dem die Kapsel 3 zuvor entnommen wurde, wieder zurückbewegt. Die Kapsel 3 wird also insbesondere radial wieder in das Magazin 5 zurückgeschoben.

Kurz bevor der Stellschieber 46 bei der radialen Rückbewegung seine Ausgangsposition wieder erreicht, erfolgt vorzugsweise sowohl ein Umschalten auf den anderen Mitnehmer 25 als auch ein Weiterdrehen des Magazins 5.

Das Weiterdrehen des Magazins 5 erfolgt beim Darstellungsbeispiel vorzugsweise dadurch, dass der Stellschieber 46 auf eine vorzugsweise geneigte Abgleitfläche 55 des Antriebselements 36 trifft, um das Antriebselement 36 nun in entgegengesetzter Umfangsrichtung wieder in die Ausgangslage zurückzubewegen, wobei aufgrund des Eingriffs der Außenzahnung des Antriebselements 36 in die Innenverzahnung des Magazins 5 das gewünschte Weiterdrehen des Magazins 5 um eine Kapsel 3 bzw. um einen gewünschten Takt bzw. Schritt von vorzugsweise 6° erfolgt, wie in Fig. 34 angedeutet.

Es ist anzumerken, dass hier auch andere konstruktive Lösungen möglich sind. Beispielsweise kann die geneigte Abgleitfläche 55 wahlweise auch am Stellschieber 46 gebildet sein und mit einem entsprechenden Ende des Antriebselements 36 beim radialen Zurückbewegen des Stellschiebers 46 abgleiten, um das Antriebselement 36 in gewünschter Weise in Umfangsrichtung zu bewegen.

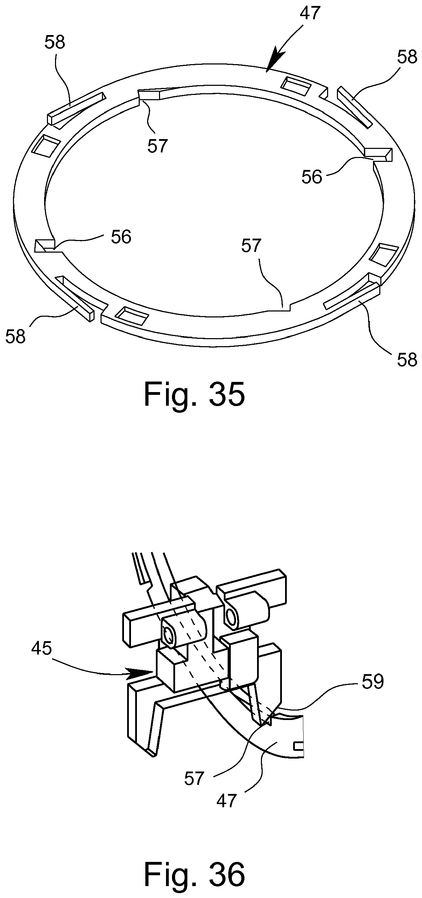

Fig. 35 zeigt einen Steuerring 47 in einer perspektivischen Ansicht. Der Steuerring 47 weist als Steuermittel vorzugweise gegenüberliegende Aussparungen 56 und gegenüberliegende Steuerflächen 57 auf.

Weiter weist der Steuerring 47 vorzugsweise eine oder mehrere Sperrzungen 58 auf, die hier über den Umfang in insbesondere 90°-Schritten verteilt sind. Die Sperrzungen 58 bilden eine Rückdrehsperre und sorgen dafür, dass der Steuerring 47 beim Öffnen und Schließen, genauer beim Aufschwenken und Zuschwenken, der Abdeckung 18 und dem damit einhergehenden Drehen der Deckel 48 jeweils nur in eine Richtung weitergedreht wird, und zwar in definierten Schritten, hier in Schritten von 90°. Dieses definierte Weiterdrehen führt dazu, dass die Steuermittel nacheinander definierte Positionen einnehmen und dass die beiden Steuerringe 47 auch ihre Stellung relativ zueinander beibehalten. Beim Darstellungsbeispiel sind die Steuerringe 47 nämlich vorzugsweise um 90° versetzt zueinander eingebaut, so dass sich immer unterschiedliche Steuermittel, also einmal eine Ausnehmung 56 und eine Steuerfläche 57 und das nächste Mal umgekehrt gegenüber liegen.

Die Sperrzungen 58 sind vorzugsweise am Außenumfang angeordnet und können mit entsprechenden Rastausnehmungen am Gehäuse 17 zusammenwirken, um die Drehsperre in einer Richtung zu realisieren.

Die Steuermittel sind vorzugsweise am Innenradius des Sperrrings 47 angeordnet.

Wenn der Stellschieber 46 radial in seine Ausgangsposition zurückbewegt wird, also beim radialen Schließen der Abdeckung 18, trifft der Stellschieber 46 kurz vor Erreichen seiner Endposition mit einer an einem unteren Ende seitlich angeordneten und geneigten Kontaktfläche 59 auf die Steuerfläche 57 und wird aufgrund der Neigung gemäß dem Prinzip der schiefen Ebene quer zur Radialbewegung, also axial bzw. zum anderen Steuerring 47 hin, wo der Stellschieber 46 mit seiner auf der anderen Seite ebenfalls angeordneten Kontaktfläche 59 in die gegenüberliegende Ausnehmung 56 einfahren kann. Durch diese Axialbewegung bzw. Querverschiebung ist das genannte Umschalten erfolgt. Hierdurch wird der Stellschieber 46 vom bisherigen Mitnehmer 25 entkoppelt und stattdessen mit dem anderen Stellschieber gekoppelt.

Beim nächsten Öffnen der Abdeckung 18 wird dann dementsprechend der andere Mitnehmer 25 radial mit in Richtung des Mundstücks 15 bzw. zur Ausgabeposition A hin bewegt und dementsprechend eine Kapsel 3 aus der anderen Ringebene der Kapseln 3 aus dem Magazin 5 heraus in die Ausgabeposition A bewegt.

Im Zusammenhang mit dem Umschalten bzw. der Querbewegung oder Axialbewegung des Stellschiebers 46 ist zu berücksichtigen, dass der Stellschieber 46 axial beweglich zwischen den beiden Schenkeln 33 der Abdeckung 18 gehalten ist, insbesondere durch Eingriff von entsprechenden, an den Schenkeln 33 angeordneten und axial nach innen abragenden Zapfen in Hülsenabschnitten am Stellschieber 46.

Fig. 36 zeigt in einem schematischen Ausschnitt den Zustand, wenn der Stellschieber 46 mit seiner Kontaktfläche 59 gerade auf den Steuerring 47 bzw. dessen Steuerfläche 57 trifft, bevor der Stellschieber 46 im weiteren Verlauf der in der Darstellung abwärts verlaufenden Radialbewegung zum Erreichen der Endstellung noch quer bzw. axial, hier nach links, verschoben wird.

**Bezugszeichenliste:**

| | | | |
|---|---|---|---|
| 1 | Inhalator | 35 | Steuerelement |
| 2 | Formulierung | 36 | Antriebselement |
| 3 | Kapsel | 37 | Führungskulisse |
| 4 | Kapselkammer | 37A | Radialabschnitt |
| 5 | Magazin | 37B | Bogenabschnitt |
| 6 | Öffnungseinrichtung | 38 | Antriebsabschnitt |
| 7 | Nadel | 39 | Aussparung |
| 8 | Nadelöffnung | 40 | vorlaufende Rippe |
| 9 | Betätigungselement | 41 | nachlaufende Rippe |
| 10 | Feder | 42 | Vorsprung |
| 11 | Einlass | 43 | Zahnstange |
| 12 | Auslass | 44 | Zahnrad |
| 13 | Ringschulter | 45 | Kopf |
| 14 | Sicherungselement | 46 | Stellschieber |
| 15 | Mundstück | 47 | Steuerring |
| 16 | Verbindungsabschnitt | 48 | Deckel |
| 17 | Gehäuse | 49 | Bewegungsschlitz |
| 18 | Abdeckung | 50 | Führungselement |
| 19 | Aufnahmekammer | 51 | Radialnut |
| 19A | Einführschräge | 52 | Bogennut |
| 20 | Fördereinrichtung | 53 | Innenverzahnung |
| 21 | Träger | 54 | Dorn |
| 22 | Halteabschnitt | 55 | Abgleitfläche |
| 23 | Führung | 56 | Ausnehmung |
| 24 | Angriffsabschnitt | 57 | Steuerfläche |
| 25 | Mitnehmer | 58 | Sperrzunge |
| 26 | Rückstellfeder | 59 | Kontaktfläche |
| 27 | Angriffselement | | |
| 28 | Ausnehmung | | |
| 29 | Eingriffselement | | |
| 30 | Federaufnahme | A | Ausgabeposition |
| 31 | Lagerabschnitt | B | Bewegung |
| 32 | Führungsschlitz | D | Drehachse |
| 33 | Schenkel | E | Hauptebene |
| 34 | Steuerkulisse | E1 | erste Kapselebene |
| 34A | Sektorabschnitt | E2 | zweite Kapselebene |
| 34B | Sektorabschnitt | H | Hauptausgaberichtung |
| 34C | Ringnutabschnitt | R | Radialbewegung |
| 34D | Haltenase | S | Schwenkbewegung |
| 34E | Unterbrechung | | |

## Patentansprüche

1. Inhalator (1) zur Inhalation einer vorzugsweise pulverförmigen Formulierung (2) aus Kapseln (3), die jeweils eine Dosis der Formulierung (2) enthalten, wobei der Inhalator (1) ein Magazin (5) mit den Kapseln (3), ein Mundstück (15) und eine dem Mundstück (15) zugeordnete Abdeckung (18) aufweist, wobei das Magazin (5) im Wesentlichen kreis-, platten- und/oder scheibenförmigen ist,
**dadurch gekennzeichnet,**
**dass** die Abdeckung (18) zum Öffnen bzw. Schließen des Mundstücks (15) relativ zum Magazin (5) radial bewegbar und schwenkbar ist, wobei durch Schwenken (S) der Abdeckung (18) das Magazin (5) von einer Kapsel (3) zur nächsten Kapsel (3) förderbar ist und wobei durch radiales Bewegen (B) der Abdeckung (18) eine Kapsel (3) aus dem Magazin (5) in radialer Richtung in eine Ausgabeposition (A) bewegbar ist, in der die Kapsel geöffnet werden kann und anschließend die Formulierung vom Patienten inhaliert werden kann.

2. Inhalator nach Anspruch 1, **dadurch gekennzeichnet, dass** der Inhalator (1) bzw. das Magazin (5) mehrere Kapselkammern (4) aufweist, die jeweils eine Kapsel (3) enthalten.

3. Inhalator nach Anspruch 2, **dadurch gekennzeichnet, dass** die Kapselkammern (4) zusammen mit der jeweils aufgenommenen Kapsel (3) einzelweise durch radiales Bewegen (R) der Abdeckung (18) aus dem Magazin (5) in die Ausgabeposition (A) bewegbar sind.

4. Inhalator nach Anspruch 2 oder 3, **dadurch gekennzeichnet, dass** die Kapselkammern (4) im Magazin (5) radial beweglich aufgenommen und einzelweise radial in die Ausgabeposition (A) bewegbar sind.

5. Inhalator nach einem der voranstehenden Ansprüche, **dadurch gekennzeichnet, dass** das Magazin (5) zur Förderung von einer Kapsel (3) bzw. Kapselkammer (4) zur nächsten Kapsel (3) bzw. Kapselkammer (4) drehbar ist, vorzugsweise durch Schwenken der Abdeckung (18).

6. Inhalator nach einem der voranstehenden Ansprüche, **dadurch gekennzeichnet, dass** die Kapseln (3) bzw. Kapselkammern (4) ringförmig mit radialer Ausrichtung angeordnet bzw. im Magazin (5) aufgenommen sind.

7. Inhalator nach einem der voranstehenden Ansprüche, **dadurch gekennzeichnet, dass** die Kapseln (3) und/oder Kapselkammern (4) abwechselnd aus verschiedenen Ebenen (E1, E2) des Magazins (5) in die Ausgabeposition (A) bzw. eine gemeinsame Aufnahmekammer (19) bewegbar sind, vorzugsweise mittels einer Einführschräge (19A).

8. Inhalator nach einem der voranstehenden Ansprüche, **dadurch gekennzeichnet, dass** der Inhalator (1) eine Fördereinrichtung (20) zur radialen Förderung der Kapseln (3) - ggf. zusammen mit einer zugeordneten Kapselkammer (4) - in radialer Richtung (R) bzw. in die Ausgabeposition (A) bzw. in die gemeinsame Aufnahmekammer (19) aufweist.

9. Inhalator nach Anspruch 8, **dadurch gekennzeichnet, dass** die Fördereinrichtung (20) innerhalb der durch die Kapseln (3) bzw. Kapselkammern (4) gebildeten ringförmigen Anordnung bzw. innerhalb des Magazins (5) angeordnet ist.

10. Inhalator nach Anspruch 8 oder 9, **dadurch gekennzeichnet, dass** die Fördereinrichtung (20) einen schieberartigen und/oder radial bewegbaren Mitnehmer (25) aufweist, vorzugsweise wobei der Mitnehmer (25) durch radiales Bewegen oder Schwenken der Abdeckung (18) radial bewegbar ist.

11. Inhalator nach einem der Ansprüche 8 bis 10, **dadurch gekennzeichnet, dass** die Fördereinrichtung (20) bzw. deren Mitnehmer (25) zwei Eingriffsbereiche oder Ausnehmungen (28) zum alternativen oder alternierenden Eingriff mit Kapseln (3) bzw. Kapselkammern (4) in verschiedenen Ebenen (E1, E2) des Magazins (5) aufweist.

12. Inhalator nach einem der voranstehenden Ansprüche, **dadurch gekennzeichnet, dass** bei Schließen der Abdeckung (18) bzw. durch eine insbesondere ununterbrochene Schwenkbewegung der Abdeckung (18) zum Schließen des Inhalators (1) eine in der Ausgabeposition (A) befindliche Kapsel (3) bzw. Kapselkammer (4) zunächst in das Magazin (5) radial zurückbewegbar und dann das Magazin (5) zur nächsten Kapsel (3) weiter förderbar bzw. drehbar ist, vorzugsweise durch eine formschlüssige Verbindung zwischen der Abdeckung (18) und dem Magazin (5).

13. Inhalator nach einem der voranstehenden Ansprüche, **dadurch gekennzeichnet, dass** eine in der Ausgabeposition (A) befindliche Kapsel (3) gegebenenfalls zusammen mit der jeweiligen Kapselkammer (4) mittels Federkraft wieder in das Magazin (5) zurückbewegbar ist.

## Claims

1. Inhaler (1) for inhalation of a formulation (2) preferably in powder form from capsules (3) which each contain a dose of the formulation (2), wherein the inhaler (1) comprises a magazine (5) having the capsules (3), a mouthpiece (15) and a cover (18) associated with the mouthpiece (15),
wherein the magazine (5) is substantially circular, plate-shaped and/or disc-shaped,
**characterised in that**
the cover (18) is radially movable and pivotable relative to the magazine (5) for opening or closing of the mouthpiece (15),
wherein by pivoting (S) of the cover (18) the magazine (5) can be conveyed from one capsule (3) to the next capsule (3) and wherein by radial movement (B) of the cover (18) a capsule (3) can be moved in the radial direction out of the magazine (5) into a discharge position (A), in which the capsule (3) can be opened and the formulation can then be inhaled by the patient.

2. Inhaler according to claim 1, **characterised in that** the inhaler (1) or the magazine (5) has a plurality of capsule chambers (4) which each contain a capsule (3).

3. Inhaler according to claim 2, **characterised in that** the capsule chambers (4) together with the capsule (3) received therein in each case can be moved individually out of the magazine (5) into the discharge position (A) by radial movement (R) of the cover (18).

4. Inhaler according to either claim 2 or claim 3, **characterised in that** the capsule chambers (4) are radially movably received in the magazine (5) and can be individually moved radially into the discharge position (A).

5. Inhaler according to any one of the preceding claims, **characterised in that** in order to be conveyed from one capsule (3) or capsule chamber (4) to the next capsule (3) or capsule chamber (4), the magazine (5) is rotatable, preferably by pivoting of the cover (18).

6. Inhaler according to any one of the preceding claims, **characterised in that** in the capsules (3) or capsule chambers (4) are arranged in a ring with a radial orientation or are received in the magazine (5).

7. Inhaler according to any one of the preceding claims, **characterised in that** the capsules (3) and/or capsule chambers (4) can be moved alternately out of different planes (E1, E2) of the magazine (5) into the discharge position (A) or into a common receiving chamber (19), preferably by means of a lead-in chamfer (19A).

8. Inhaler according to any one of the preceding claims, **characterised in that** the inhaler (1) comprises a conveying device (20) for radially conveying the capsules (3) - optionally together with an associated capsule chamber (4) - in the radial direction (R) or into the discharge position (A) or into the common receiving chamber (19).

9. Inhaler according to claim 8, **characterised in that** the conveying device (20) is arranged inside the annular arrangement formed by the capsules (3) or capsule chambers (4) or is arranged inside the magazine (5).

10. Inhaler according to either claim 8 or claim 9, **characterised in that** the conveying device (20) has a slide-like and/or radially movable driver (25), preferably wherein the driver (25) is radially movable by radial movement or pivoting of the cover (18).

11. Inhaler according to any one of claims 8 to 10, **characterised in that** the conveying device (20) or the drivers (25) thereof has/have two engagement regions or cutouts (28) for alternative or alternating engagement with capsules (3) or capsule chambers (4) in different planes (E1, E2) of the magazine (5).

12. Inhaler according to any of the preceding claims, **characterised in that**, during closing of the cover (18) or by an in particular uninterrupted pivoting movement of the cover (18) for closing the inhaler (1) a capsule (3) or capsule chamber (4) located in the discharge position can first of all be moved back radially into the magazine (5) and then the magazine (5) can be further conveyed or rotated to the next capsule (3), preferably by a positively engaged connection between the cover (18) and the magazine (5).

13. Inhaler according to any one of the preceding claims, **characterised in that** a capsule (3) located in the discharge position (A) can be moved back into the magazine (5) again, optionally together with the respective capsule chamber (4), by means of spring force.

## Revendications

1. Inhalateur (1) pour l'inhalation d'une formulation (2) de préférence pulvérulente à partir de capsules (3), qui contiennent respectivement une dose de la formulation (2), dans lequel l'inhalateur (1) présente un magasin (5) avec les capsules (3), un embout (15) et un capuchon (18) associé à l'embout (15), dans lequel le magasin (5) est sensiblement en forme de cercle, de plaque et/ou de disque,
**caractérisé en ce**
**que** le capuchon (18) est pivotant et déplaçable radialement par rapport au magasin (5) pour ouvrir et fermer l'embout (15), dans lequel le magasin (5) peut être déplacé d'une capsule (3) à la prochaine capsule (3) par pivotement (S) du capuchon (18) et dans lequel une capsule (3) est déplaçable par déplacement radial (B) du capuchon (18) hors du magasin (5) dans la direction radiale dans une position de distribution (A), dans laquelle la capsule peut être ouverte et ensuite la formulation peut être inhalée par le patient.

2. Inhalateur selon la revendication 1, **caractérisé en ce que** l'inhalateur (1) ou le magasin (5) présente plusieurs chambres de capsule (4), qui contiennent respectivement une capsule (3).

3. Inhalateur selon la revendication 2, **caractérisé en ce que** les chambres de capsule (4) sont déplaçables conjointement avec la capsule respectivement reçue (3) une à une par déplacement radial (R) du capuchon (18) hors du magasin (5) dans la position de distribution (A).

4. Inhalateur selon la revendication 2 ou 3, **caractérisé en ce que** les chambres de capsule (4) sont reçues radialement mobiles dans le magasin (5) et déplaçables une à une radialement dans la position de distribution (A).

5. Inhalateur selon l'une quelconque des revendications précédentes, **caractérisé en ce que** le magasin (5) est rotatif pour le transport d'une capsule (3) ou chambre de capsule (4) à la prochaine capsule (3) ou chambre de capsule (4), de préférence par pivotement du capuchon (18).

6. Inhalateur selon l'une quelconque des revendications précédentes, **caractérisé en ce que** les capsules (3) ou chambres de capsule (4) sont agencées de manière annulaire avec orientation radiale ou sont reçues dans le magasin (5).

7. Inhalateur selon l'une quelconque des revendications précédentes, **caractérisé en ce que** les capsules (3) et/ou chambres de capsule (4) sont déplaçables alternativement à partir de différents niveaux (E1, E2) du magasin (5) dans la position de distribution (A) ou une chambre de réception commune (19), de préférence au moyen d'un chanfrein d'introduction (19A).

8. Inhalateur selon l'une quelconque des revendications précédentes, **caractérisé en ce que** l'inhalateur (1) présente un dispositif de transport (20) pour le transport radial des capsules (3) (le cas échéant conjointement avec une chambre de capsule associée (4)) dans la direction radiale (R) ou dans la position de distribution (A) ou dans la chambre de réception commune (19).

9. Inhalateur selon la revendication 8, **caractérisé en ce que** le dispositif de transport (20) est agencé à l'intérieur de l'agencement annulaire formé par les capsules (3) ou chambres de capsule (4) ou à l'intérieur du magasin (5).

10. Inhalateur selon la revendication 8 ou 9, **caractérisé en ce que** le dispositif de transport (20) présente un entraîneur (25) de type coulisseau et/ou radialement mobile, de préférence dans lequel l'entraîneur (25) est radialement mobile par pivotement ou déplacement radial du capuchon (18).

11. Inhalateur selon l'une quelconque des revendications 8 à 10, **caractérisé en ce que** le dispositif de transport (20) ou son entraîneur (25) présente deux zones de mise en prise ou évidements (28) pour la mise en prise alternative ou alternante avec des capsules (3) ou chambres de capsule (4) à différents niveaux (E1, E2) du magasin (5).

12. Inhalateur selon l'une quelconque des revendications précédentes, **caractérisé en ce que** lors de la fermeture du capuchon (18) ou par un mouvement de pivotement en particulier ininterrompu du capuchon (18) pour la fermeture de l'inhalateur (1), une capsule (3) se trouvant dans la position de distribution (A) ou chambre de capsule (4) peut d'abord être reculée radialement dans le magasin (5) et ensuite le magasin (5) est transportable ou rotatif vers la prochaine capsule (3), de préférence par une liaison par correspondance de forme entre le capuchon (18) et le magasin (5).

13. Inhalateur selon l'une quelconque des revendications précédentes, **caractérisé en ce qu'**une capsule (3) se trouvant dans la position de distribution (A) le cas échéant conjointement avec la chambre de capsule (4) respective peut être reculée à nouveau dans le magasin (5) par force de ressort.
